(19) Europäisches Patentamt / European Patent Office / Office européen des brevets

(11) **EP 4 186 412 A1**

(12) **EUROPEAN PATENT APPLICATION**

(43) Date of publication:
**31.05.2023   Bulletin 2023/22**

(21) Application number: **22209800.6**

(22) Date of filing: **28.11.2022**

(51) International Patent Classification (IPC):
*A61B 3/103* (2006.01)

(52) Cooperative Patent Classification (CPC):
**A61B 3/103**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.11.2021   JP 2021193658**
**29.11.2021   JP 2021193659**

(71) Applicant: **Nidek Co., Ltd.**
**Aichi 443-0038 (JP)**

(72) Inventor: **TAKII, Michihiro**
**Gamagori-shi, Aichi, 4430038 (JP)**

(74) Representative: **Hoefer & Partner Patentanwälte mbB**
**Pilgersheimer Straße 20**
**81543 München (DE)**

(54) **EYE EXAMINATION DEVICE, ATTACHMENT, AND EYE EXAMINATION PROGRAM**

(57)    An eye examination device (1) includes a first objective measurement means (40, 50) configured to objectively measure eye refractive power of at least one of subject eyes (E) using a method different from a photorefraction method, a second objective measurement means (90, 100) configured to objectively measure eye refractive power of at least one of the subject eyes using the photorefraction method, and a controller (130) configured to control at least one of the first objective measurement means or the second objective measurement means to acquire the eye refractive power.

FIG. 1

**Description**

TECHNICAL FIELD

The present disclosure relates to an eye examination device, an attachment, and an eye examination program.

BACKGROUND ART

As an example of information on a subject eye, an eye examination device that objectively acquires the eye refractive power of the subject eye is known. For example, by projecting a measurement light flux onto the fundus of the subject eye and receiving the fundus-reflected light flux, the eye refractive power of the subject eye can be measured (see JP2006-187483A).

Such an eye examination device includes a stationary type as in JP2006-187483Aand a hand-held type as in JP2012-183123A.

Acquisition of eye refractive power is required in various situations in accordance with the purpose and the application of an examiner or an examinee. For example, there are situations where exact precision is required, situations where simple measurement is required, situations where efficient measurement is required, and the like. For this reason, it is desired to cope with the measurement in accordance with the situation.

SUMMARY OF INVENTION

A technical object of the present disclosure is to provide an eye examination device configured to acquire eye refractive power of a subject eye in accordance with a situation, an attachment, and an eye examination program used in the eye examination device.

In order to solve the above problems, the present disclosure has a configuration as follows.

An eye examination device including:

a first objective measurement unit configured to objectively measure eye refractive power of at least one of subject eyes using a method different from a photorefraction method;
a second objective measurement unit configured to objectively measure eye refractive power of at least one of the subject eyes using the photorefraction method; and
a controller configured to control at least one of the first objective measurement unit or the second objective measurement unit to acquire the eye refractive power.

An attachment to be mounted to an eye examination device including a first objective optical system configured to objectively acquire information on a subject eye, the attachment including:
a conversion optical system configured to convert the first objective optical system into a second objective optical system configured to objectively measure eye refractive power of the subject eye using the photorefraction method.

An eye examination device to which the attachment is capable of being mounted, the eye examination device including:

a mounting portion to which the attachment is to be mounted; and
a connection portion configured to electrically connect the eye examination device and the attachment, in which the attachment becomes available in a case where the connection portion electrically connects the eye examination device and the attachment.

A non-transitory computer-readable storage medium storing an eye examination program used in an eye examination device including a first objective measurement unit configured to objectively measure eye refractive power of a subject eye using a method different from a photorefraction method, and a second objective measurement unit configured to objectively measure eye refractive power of the subject eye using the photorefraction method, the eye examination program including instructions which, when executed by a processor of the eye examination device, cause the eye examination device to:
control at least one of the first objective measurement unit or the second objective measurement unit to acquire the eye refractive power.

BRIEF DESCRIPTION OF DRAWINGS

FIG. 1 is an external diagram of a state where a hand-held eye examination device is mounted to an attachment.
FIG. 2 is an external view of a state where the attachment is removed from the hand-held eye examination device.
FIG. 3 is a schematic configuration diagram of a first measurement unit.
FIG. 4 is a schematic configuration diagram of a second measurement unit.
FIG. 5 is an example of a display unit in a first measurement mode.
FIG. 6 is an example of the display unit in a second measurement mode.
FIG. 7 is a diagram for illustrating a photo refraction method.
FIG. 8 is an external view of a stationary eye examination device.
FIG. 9 is a schematic configuration diagram of a measurement unit.

## DESCRIPTION OF EMBODIMENTS

### <Outline>

An outline of an eye examination device and an attachment to be mounted thereto according to an embodiment of the present disclosure will be described. The items classified in < > below may be used independently or in conjunction with each other. It is assumed that the term "conjugated" in the present embodiment is not necessarily limited to a perfect conjugated relationship, but includes "substantially conjugated". That is, the term "conjugated" in the present embodiment also includes a case where each unit is disposed to be shifted from the perfectly conjugated position within a range allowed in relation to the technical significance of each unit.

### <Eye Examination Device>

The eye examination device in the present embodiment may be a hand-held eye examination device or a stationary eye examination device. The eye examination device may include at least two objective measurement means and be configured to measure eye refractive power of a subject eye using different methods. By controlling at least one of a plurality of objective measurement means, it is possible to acquire the eye refractive power of the subject eye in response to various situations.

The eye examination device may include a first objective measurement means (for example, a first measurement unit 40 and a measurement unit 260) configured to objectively measure the eye refractive power of a subject eye using a method different from a photorefraction method. The first objective measurement means only needs to be configured to include at least a first objective measurement optical system (for example, a first measurement optical system 50) as a portion configuring the first objective measurement means.

The eye examination device may also include a second objective measurement means (for example, a second measurement unit 90 and a measurement unit 260) configured to objectively measure eye refractive power of the subject eye using a photorefraction method. The second objective measurement means only needs to be configured to include at least a second objective measurement optical system (for example, a second measurement optical system 100 and a second measurement optical system 300) as a portion configuring the second objective measurement means.

In addition, the eye examination device may include controller (for example, a controller 130) configured to control at least one of the first objective measurement means and the second objective measurement means to acquire the eye refractive power.

### <First Objective Measurement Means>

For example, the first objective measurement means may be configured to project a measurement light flux onto a fundus of one of subject eyes to measure the eye refractive power using a method different from the photorefraction method. In this case, although the first objective measurement means requires more precise alignment than the second objective measurement means, the measurement accuracy can be improved.

For example, the first objective measurement means may be an objective measurement means configured to project a measurement light flux onto the fundus of the subject eye, and to cause a first detector to receive a reflected light flux that is the measurement light flux reflected from the fundus. At this time, the first detector may be disposed at a fundus conjugate position of the subject eye.

Further, for example, the first objective measurement means may be an objective measurement means configured to project a pattern index, as a measurement light flux, onto the fundus of the subject eye, and to cause a first detector to receive the reflected light flux that is the measurement light flux reflected from the fundus. In this case, the controller may be configured to acquire the eye refractive power based on the reflected light flux received by the first detector. As an example, the reflected light flux may be extracted as a ring image to acquire the eye refractive power based on the ring image. Further, as an example, the reflected light flux may be detected by a Shack-Hartmann sensor to acquire the eye refractive power. That is, the first objective measurement means may be an imaging-type objective measurement means. In other words, the first objective measurement means may be an objective measurement means configured to measure the eye refractive power by applying a refractometer method.

Further, for example, as the first objective measurement means, there may be an objective measurement means configured to project measurement light fluxes from at least two light sources onto the fundus of the subject eye, and to cause the first detector to receive the reflected light fluxes that are the measurement light fluxes reflected from the fundus. In this case, spot light may be projected onto the subject eye. Further, in this case, the controller may be configured to acquire the eye refractive power based on a matching state of the two reflected light fluxes received by the first detector. That is, the first objective measurement means may be a matching objective measurement means.

Further, for example, as the first objective measurement means, there may be an objective measurement means configured to scan the fundus of the subject eye with a measurement light flux, and to cause the first detector to receive the reflected light flux that is the measurement light flux reflected from the fundus. In this case, slit light may be projected onto the subject eye. Further, in this case, the controller may be configured to acquire the eye refractive power based on a phase difference signal from the first detector. That is, the first objective measurement means may be an objective measurement means configured to measure the eye refractive power using a phase difference method.

<Second Objective Measurement Means>

For example, the second objective measurement means may be configured to project a measurement light flux onto fundi of both of the subject eyes to measure the eye refractive power using the photorefraction method. In this case, although the second objective measurement means has lower measurement accuracy than the first objective measurement means, it is possible to obtain the measurement results simply and efficiently.

For example, the second objective measurement means may be an objective measurement means configured to project a measurement light flux onto the fundus of the subject eye, and to cause a second detector to receive a reflected light flux that is the measurement light flux reflected from the fundus. At this time, the second detector may be disposed at a pupil conjugate position of the subject eye.

Further, for example, the second objective measurement means may be an objective measurement means configured to project a non-pattern index, as a measurement light flux, onto the fundus of the subject eye, and to cause the second detector to receive the reflected light flux that is the measurement light flux reflected from the fundus. In this case, the controller may be configured to acquire the eye refractive power based on a state of the light flux, which has been received by the second detector, at the pupil of the subject eye. As an example, the eye refractive power may be acquired based on the ratio of the light flux at the pupil. That is, the second objective measurement means may be an objective measurement means configured to measure the eye refractive power by a shadow test (skiascopy or retinoscopy).

The second objective measurement means will be described in detail. The second objective measurement means may include a light projection optical system (for example, a light projection optical system 110 and a light projection optical system 310) and a light reception optical system (for example, a light reception optical system 120 and a light reception optical system 320). For example, the light projection optical system and the light reception optical system may be configured by separate optical members, or may be configured by sharing at least some optical members.

The light projection optical system only needs to be configured to include at least a measurement light source. For example, the light projection optical system includes a plurality of measurement light sources (for example, a measurement light source 111 and a measurement light source 311) disposed in a meridian direction (radial direction) with reference to an optical axis center. The light projection optical system irradiates the fundus of the subject eye with measurement light fluxes emitted from the plurality of measurement light sources. Each of the plurality of measurement light sources may be independently controlled. For example, the lighting and extinguishing of each measurement light source, the adjustment of the amount of light, and the like may be controlled independently. Further, the plurality of measurement light sources may be disposed separately from each other in at least three meridian directions with respect to the optical axis center. For example, by disposing the measurement light sources in at least the three meridian directions, it is possible to measure the eye refractive power including spherical power, cylindrical power, an astigmatic axis angle, and the like. The meridian directions in which the measurement light sources are disposed can be any number of meridian directions (for example, one meridian direction, two meridian directions, three meridian directions, four meridian directions, and the like). At least one measurement light source only needs to be disposed in the meridian direction.

For example, the light projection optical system may include an objective optical system. The objective optical system irradiates the fundus of the subject eye with measurement light fluxes emitted from a plurality of measurement light sources. The objective optical system may include one or more optical members configured to project a measurement light flux towards the subject eye.

The light reception optical system only needs to be configured to include at least a detector. For example, the light reception optical system may be configured to cause the detector to receive the reflected light flux that is the measurement light flux reflected from the fundus of the subject eye (for example, an imaging element 122 and an imaging element 323).

For example, the light reception optical system may include an objective optical system (for example, a wide-angle lens 121 and a wide-angle lens 321). The objective optical system is configured to guide the reflected light flux that is the measurement light flux reflected from the fundus of the subject eye to the detector. The objective optical system may include one or more optical members configured to guide the light flux reflected from the fundus of the subject eye to the detector.

In the present embodiment, since the first objective measurement means is configured to measure the eye refractive power of one of the subject eyes using the method different from the photorefraction method, and the second objective measurement means is configured to measure both of the subject eyes using the photorefraction method, each objective measurement means differs in at least one of measurement accuracy and measurement easiness (as an example, alignment easiness). For example, the first objective measurement means has higher measurement accuracy and lower measurement easiness (in other words, more difficult) than the second objective measurement means. The second objective measurement means has lower measurement accuracy and higher measurement easiness (in other words, simpler) than the first measurement means. Therefore, it is possible to use the two measurement methods according to the situation.

Further, in the present embodiment, since the first objective measurement means is set to a fundus conjugate system, and the second objective measurement means is set to a pupil conjugate system, it is possible to easily select the measurement accuracy and measurement easiness corresponding to the subject eye by switching between the two measurement methods.

Further, in the present embodiment, since the first objective measurement means is configured to acquire the eye refractive power based on at least one of the ring image, the matching state of the reflected light flux, the phase difference signal, and the like, it is possible to obtain information on many meridian directions. As a result, the eye refractive power of the eye can be measured more accurately than the second objective measurement means.

<Double Use of Objective Measurement Means>

The first objective measurement means and the second objective measurement means may be provided independently of each other, or at least some optical members may be shared in the optical system configuring each objective measurement means.

The first objective measurement means may include the light projection optical system configured to project light toward the anterior segment of the subject eye. For example, the light projection optical system may be an illumination optical system configured to illuminate the anterior segment. Further, for example, the light projection optical system may be an index projection optical system (for example, an index projection optical system 70) configured to project an alignment index light flux onto the subject eye. Further, for example, the light projection optical system may be an index projection optical system configured to project an index light flux for measuring a corneal shape onto the subject eye. The light projection optical system only needs to be configured to include at least a light source.

The light source of the light projection optical system provided in the first objective measurement means may also be used as the measurement light source of the second objective measurement means. That is, a light source for illuminating the anterior segment of the subject eye or a light source for projecting an index light flux onto the anterior segment of the subject eye may be also used as a measurement light source for projecting the measurement light flux onto the fundus in photorefraction measurement of the subject eye. As a result, it is possible to acquire the eye refractive power based on different measurement methods with a simple configuration, without providing dedicated light sources for the first objective measurement means and the second objective measurement means.

In the second objective measurement means, the plurality of measurement light sources can be disposed in the meridian direction with reference to the optical axis center of the light projection optical system, and acquire the eye refractive power based on the movement of light when each measurement light source is turned on in order. For this reason, the light source of the light projection optical system in the first objective measurement means may be configured to identify the meridian direction by using a light source configured to emit a point-like or line-like light flux.

The first objective measurement means may include an anterior segment observation optical system configured to capture an image of the anterior segment of the subject eye. For example, the anterior segment observation optical system only needs to be an optical system configured to capture an image of the anterior segment. The anterior segment observation optical system only needs to be configured to include at least a detector.

A detector of the anterior segment observation optical system provided in the first objective measurement means may also be used as the detector of the second objective measurement means. That is, a detector configured to capture an image of the anterior segment of the subject eye may be used as a detector (second detector) configured to receive the reflected light flux that is the measurement light flux reflected from the fundus in photo refraction measurement of the subject eye. This makes it possible to acquire the eye refractive power based on different measurement methods with a simple configuration, without providing dedicated detectors for the first objective measurement means and the second objective measurement means.

The eye examination device may have a configuration in which only the light source of the first objective measurement means is also used as the light source of the second objective measurement means. The eye examination device may have a configuration in which only the detector of the first objective measurement means is also used as the detector of the second objective measurement means. The eye examination device may have a configuration in which both the light source of the first objective measurement means and the detector of the first objective measurement means is also used as the light source of the second objective measurement means and the detector of the second objective measurement means, respectively.

<First Alignment Means>

The eye examination device may include first alignment means configured to capture an image of the subject eye and to adjust a positional relationship between the subject eye and the first objective measurement means. For example, the first alignment means may be used in a measurement method different from the photorefraction method. Also, for example, the first alignment means may be configured to capture an image of the anterior segment of the subject eye in a narrow range to adjust the positional relationship. In this case, an operation distance from the subject eye to the first objective measurement means may be set to be shorter than an operation distance from the subject eye to the second objective measurement means.

The first alignment means only needs to include at least a light source and a detector. For example, the first alignment means may include an index projection optical system (for example, an index projection optical system 70). In this case, an index light flux may be projected onto cornea of the subject eye, and an alignment index image based on the reflected light flux that is the index light flux reflected from the cornea may be detected. The anterior segment observation optical system (for example, an anterior segment observation optical system 80) configured to capture an image of the anterior segment of the subject eye may also have a function of detecting the alignment index image. That is, the first alignment means may be an alignment optical system that includes an index projection optical system and an anterior segment observation optical system.

For example, the first alignment means may be configured to finely adjust the relative positional relationship of the first objective measurement means with respect to the subject eye by using the alignment index image. That is, more precise alignment may be performed by using the first alignment means than that in a case by using the second alignment means.

<Second Alignment Means>

The eye examination device may include a second alignment means configured to capture the subject eye at a wider angle than an angle in the first alignment means and to adjust the positional relationship between the subject eye and the second objective measurement means. For example, the first alignment means may be used in the photorefraction measurement. Further, for example, the second alignment means may be configured to capture the anterior segment of the subject eye in a wide range to adjust the positional relationship between the subject eye and the second objective measurement means. In this case, an operation distance from the subject eye to the second objective measurement means may be set to be longer than an operation distance from the subject eye to the first objective measurement means.

For example, the second alignment means only needs to at least include a detector. For example, the second alignment means may be an anterior segment observation optical system (for example, the anterior segment observation optical system 80 and a light reception optical system 320) configured to capture the anterior segment of the subject eye. For example, the second alignment device may roughly adjust the relative positional relationship of the second objective measurement means with respect to the subject eye by using an anterior segment observation image of the subject eye. That is, rougher alignment may be performed by using the second alignment means than a case using the first alignment means.

<Controller>

The controller may be configured to acquire the eye refractive power using the first objective measurement means using a method different from the photorefraction method. Further, the controller may be configured to acquire the eye refractive power using the second objective measurement means using a photorefraction method. In addition, the controller may be configured to acquire both the eye refractive power using the first objective measurement means and the eye refractive power using the second objective measurement means. The controller may be configured to acquire an average value or the like of the eye refractive power obtained using the first objective measurement means and the eye refractive power obtained using the second objective measurement means, as the eye refractive power.

The controller may be configured to set one of a first measurement mode using the first objective measurement means and a second measurement mode using the second objective measurement means based on a switching signal of the measurement mode for measuring the eye refractive power of the subject eye. This makes it possible to smoothly set the measurement mode and easily start the measurement using different measurement methods.

For example, the switching signal may be output in response to an operation of operation means (for example, an operation unit 11 and an operation unit 210) by an examiner. As an example, the switching signal may be output in response to a selection of the measurement mode or the like. For example, the switching signal may be output in accordance with a measurement program that automatically proceeds measurement of the subject eye. As an example, the switching signal may be output in at least one of the following cases: a case where it is not possible to obtain a favorable result, a case where the measurement takes a predetermined time or longer, and the like in one measurement mode.

The configuration in which the eye examination device includes the first objective measurement means and the second objective measurement means has been exemplified. Any objective measurement means may be provided as the attachment to be mounted to the eye examination device. For example, the eye examination device may include the first objective measurement means, and the attachment may include the second objective measurement means. Even with such a configuration, the two objective measurement means can share a portion thereof.

<Attachment>

In the present embodiment, the eye examination device includes the first objective measurement means, and, by mounting an attachment to the eye examination device, it may be possible to objectively measure the eye refractive power of the subject eye using the photorefraction method.

[Conversion Optical System]

The attachment may include a conversion optical system (for example, a light projection optical system 110 and a light reception optical system 120) configured to convert a first objective optical system of the eye examination device into a second objective optical system (for example, a second measurement optical system 100) configured to objectively measure the eye refractive power of the subject eye using the photorefraction method. For example, in a case where the attachment is mounted to the eye examination device, the conversion optical system is disposed in an optical path of the first objective optical system, and the conversion optical system is used as a portion of the first objective optical system to convert the first objective optical system into the second objective optical system. For example, the configuration of the first objective optical system is converted into the second objective optical system such that the fundus of the subject eye is irradiated with the measurement light flux and the ratio (light movement) of the reflected light flux from the fundus in the pupil can be observed. Depending on whether the attachment is mounted or dismounted, it is possible to acquire the eye refractive power of the subject eye using a method different from the photorefraction method and the eye refractive power of the subject eye using the photorefraction method.

The conversion optical system may include at least one of a plurality of measurement light sources (for example, the measurement light source 111) disposed in the meridian direction with respect to the optical axis center of the conversion optical system, and a wide-angle lens (for example, the wide-angle lens 121) configured to widen an image capturing angle-of-view of the second objective optical system in a case where the attachment is mounted compared with an image capturing angle-of-view of the first objective optical system in a case where the attachment is not mounted.

For example, the conversion optical system may include only a plurality of measurement light sources. In this case, the wide-angle lens may be removably provided in the optical path of the first objective optical system. It is possible to easily convert the measurement method of the subject eye in a manner that the conversion optical system is added, using the attachment, to the first objective optical system of the eye examination device, and a wide-angle lens is inserted in the optical path of the first objective optical system.

Further, for example, the conversion optical system may include only a wide-angle lens. In this case, the plurality of measurement light sources may be provided in the optical path of the first objective optical system.

tem. It is possible to easily convert the measurement method of the subject eye in a manner that the first objective optical system of the eye examination device includes the plurality of measurement light sources, and a wide-angle lens is added, using the attachment, to the measurement light source. As described above, the plurality of measurement light sources used in the photorefraction measurement of the subject eye may also be used as light sources for other optical systems provided in the eye examination device, or may be provided exclusively.

Further, for example, the conversion optical system may include both the plurality of measurement light sources and the wide-angle lens. It is possible to easily convert the measurement method of the subject eye by adding the conversion optical system, using the attachment, to the first objective optical system of the eye examination device.

With such a disposition of the wide-angle lens, the image capturing angle-of-view of the first objective optical system in the eye examination device is switched between an image capturing angle-of-view in a case where the eye refractive power of the subject eye is measured using the method different from the photorefraction method and an image capturing angle-of-view in a case where the eye refractive power of the subject eye is measured using the photorefraction method. For example, in the method different from the photorefraction method, an image of the subject eye in a narrow range is captured, and the measurement accuracy is improved as compared with the photorefraction method. Further, for example, in the photorefraction method, an image of the subject eye in a wide range is captured, and the measurement easiness is improved as compared with the method different from the photorefraction method. The first objective optical system may include an anterior segment observation optical system, and the image capturing angle-of-view of the anterior segment may be switched by the disposition of the wide-angle lens.

<Distance Measurement Means>

The attachment may include distance measurement means (for example, a distance measurement unit 31) configured to measure the distance from the subject eye to the eye examination device. For example, the distance measurement means may be configured to measure the distance from the subject eye to the attachment, or to measure the distance from the subject eye to a housing (for example, a housing portion 20). Further, for example, the distance measurement means may be configured to measure the distance from the subject eye to the attachment, and add the length of the attachment to the measured distance, thereby measuring the distance from the subject eye to the housing. The distance from the subject eye to the eye examination device may be represented by the distance to a predetermined member in the eye examination device. For example, the predetermined member may be an attachment, the surface (front surface, rear surface, and the like) of the housing, an optical member of the conversion optical system or the first objective optical system, or the like.

The distance measurement means only needs to be configured to measure the distance between the subject eye and the eye examination device. For example, the distance measurement means may be configured to project an index light flux onto the cornea of the subject eye and to measure the distance using an alignment index image based on a reflected light flux that is the index light flux reflected from the cornea. That is, the distance measurement means may be an alignment optical system. Further, for example, the distance measurement means may be configured to emit an optical signal toward the subject eye and to detect a reflected signal that is the optical signal reflected from the subject eye to measure the distance. That is, the distance measurement means may be an optical detector. Further, for example, the distance measurement means may be configured to emit an ultrasonic wave toward the subject eye and to detect a reflected wave that is the ultrasonic wave reflected from the subject eye to measure the distance. That is, the distance measurement means may be an ultrasonic detector. Optical systems and detectors different from the above examples may be used.

It is preferable to use an ultrasonic detector as the distance measurement means. In this case, the distance measurement means may include an ultrasonic transmission unit (for example, an ultrasonic transmission unit 31a) configured to transmit an ultrasonic wave toward the subject eye, and an ultrasonic reception unit (for example, an ultrasonic reception unit 31b) configured to receive an ultrasonic wave reflected from the subject eye. As described above, in the photorefraction measurement of the subject eye, the image capturing angle-of-view of the objective optical system is widened compared with that of the first objective optical system. In a case of a configuration using ultrasonic waves, it is easy to measure distance without an influence due to a change in the image capturing angle-of-view.

In a case of a configuration in which the measurement method of the eye refractive power of the subject eye can be switched by such attaching or detaching of the attachment to and from the eye examination device, the eye examination device may be provided with a mounting means, a connection means, a detection means, and the like.

<Mounting Means>

The eye examination device may include mounting means (for example, a mounting portion 23) to which the attachment is to be mounted. The mounting means only needs to be configured to fix the attachment. For example, the mounting means may include a fitting mechanism configured to be fitted with the attachment. In this case, the mounting means may have either the structure of a convex portion or a concave portion, and the attachment may have the structure of the other. Further, for example, the mounting means may include a coupling mechanism configured to couple the attachment. In this case, the mounting means and the attachment may contain a ferromagnetic material and be magnetically coupled to each other. The mounting means may include a mechanism different from the above example, or may include a mechanism obtained by combining at least one of a fitting mechanism, a coupling mechanism, a mechanism different from the fitting mechanism and the coupling mechanism, and the like.

<Connection Means>

The eye examination device may include connection means (for example, an electrical connection portion 24) configured to electrically connect the eye examination device and the attachment. The attachment is used in a case where the connection portion electrically connects the eye examination device and the attachment. The connection means may be configured to transmit and receive electrical signals via wireless communication. Further, the connection means may be configured to transmit and receive electrical signals via wired communication. As an example, the connection means may be a connector or the like.

The connection means may be configured to electrically connect the eye examination device and the attachment in conjunction with mounting of the attachment to the eye examination device. For example, the connection means may be configured such that the terminals of a connector and terminal holes come into direct contact with each other in conjunction with mounting of the attachment. In this case, the connection means may include either the structure of the terminal or the terminal hole, and the attachment may include the structure of the other.

Further, the connection means may be configured to electrically connect the eye examination device and the attachment without conjunction with mounting of the attachment to the eye examination device. For example, the connection means and the attachment may be configured to come into indirect contact with each other via a cable or the like. In this case, after mounting the attachment, a connector may be separately connected to the attachment and the connection means. The attachment and the connection means may be electrically connected to each other by fixing a cable to the attachment and bringing a connector of the cable into contact with the connection means. The attachment and the connection means may be electrically connected to each other by fixing a cable to the connection means (in other words, the cable may be used as a portion of the connection means), and brining the connector of the cable into contact with the attachment.

<Detector>

The eye examination device may include detector (for example, a mounting detector 23a and a connection detector 25) configured to detect at least one of mounting of the attachment to the eye examination device and an electrical connection between the eye examination device and the attachment. The detector only needs to be configured to detect at least whether or not the eye examination device is in contact with the attachment in a case where the attachment is mounted to the eye examination device. In this case, as the detector, a contact-type detector (as an example, a physical sensor and the like), a non-contact-type detector (as an example, an optical sensor, a magnetic sensor, and the like), or the like may be used. Further, the detector only needs to be configured to detect at least whether or not the eye examination device and the attachment are electrically connected. In this case, a non-contact detector (as an example, a current sensor, a voltage sensor, and the like) or the like may be used as the detector.

<Controller>

Even in a case where the attachment is attached to or detached from the eye examination device, the eye examination device can use the configuration of the controller described in the above section <Eye Examination Device>. For example, the controller is configured to control at least one of the first objective optical system of the eye examination device and the conversion optical system of the attachment to acquire the eye refractive power of the subject eye. Furthermore, for example, the controller may be configured to perform various controls associated with attachment and detachment of the attachment.

The controller may be configured to control an action of the eye examination device based on the detection signal from the detector. For example, the controller may be configured to perform control to change the action of the eye examination device, in a case where a detection signal is obtained for either mounting of the attachment to the eye examination device or an electrical connection between the eye examination device and the attachment. Further, the controller may be configured to perform control to change the action of the eye examination device, after both detection signals for mounting of the attachment and the electrical connection are obtained.

For example, the controller may be configured to automatically switch a mode from a first measurement mode using the first objective optical system to a second measurement mode using the second objective optical system (in other words, the first objective optical system and the conversion optical system), based on the detection signal from the detector. For example, the controller may be configured to automatically switch the mode based on a switching signal accompanying the operation of the operation means by the examiner. In the above cases, the controller may be configured to control at least one of display means (for example, a display unit 22), a sound generation means (as an example, a speaker or the like), a notification means (as an example, a lamp or the like), and the like to output notification information for a notification indicating that the setting has been changed from the first measurement mode to the second measurement mode.

The controller may output leading information for leading a setting change from the first measurement mode using the first objective optical system to the second measurement mode using the second objective optical system, based on the detection signal from the detector. For example, the leading information may be information for leading an examiner to perform an action. As an example, the leading information may be information for leading the examiner to perform the setting change from the first measurement mode to the second measurement mode, an operation of a switch or the like for performing switching between the first measurement mode and the second measurement mode, ensuring of an operation distance between the subject eye and the eye examination device, and the like. The leading information may be a combination of the above types of information, or may be information different from the above types of information.

In this case, the controller may be configured to control the display means to display the leading information on the display means. Further, for example, the controller may be configured to control the sound generation means to cause the sound generation means to generate the leading information as sound. Further, for example, the controller may be configured to control the notification means to display the leading information by lighting or blinking of the notification means. The controller may be configured to perform control of a combination of the above controls, or may perform control different from the above controls.

In the eye examination device in the present embodiment, the first objective optical system is set to an optical system configured to objectively measure the eye refractive power of the subject eye using the method different from the photorefraction method, but the present embodiment is not limited to this. The first objective optical system may be any optical system configured to objectively acquire information on the subject eye. As an example, the first objective optical system may be an optical system configured to capture the anterior segment of the subject eye and to acquire anterior segment observation image data of the subject eye, corneal shape data of the subject eye, and the like. Further, the first objective optical system may be an optical system configured to capture the fundus of the subject eye and to acquire fundus tomographic image data of the subject eye, fundus front image data of the subject eye, and the like. In addition, the first objective optical system may be an optical system configured to measure the subject eye and to acquire the optical characteristic of the subject eye, the eye refractive power of the subject eye, the binocular vision function (tilt amount, stereoscopic vision function, and the like), contrast sensitivity, and the like.

More specifically, the eye examination device may be at least one of an optical coherence tomography device, a scanning laser ophthalmoscope, a fundus camera, an intraocular pressure measurement device, an eye axial length measurement device, a corneal shape measurement device, a corneal curvature measurement device, an ultrasonic optometric device, an eye refractive power measurement device, and the like. Alternatively, the eye examination device may be a composite device of the above devices.

For example, by attaching the attachment to such an eye examination device, it is possible to convert the first objective optical system configured to objectively acquire information on the subject eye into the second objective optical system configured to objectively measure the eye refractive power of the subject eye using the photorefraction method. Therefore, it is possible to easily acquire the information on the subject eye and the eye refractive power of the subject eye using the photorefraction method, by using one eye examination device.

In addition, in the eye examination device in the present embodiment, the second objective optical system is set to an optical system configured to objectively measure the eye refractive power of the subject eye using the photorefraction method, but the present embodiment is not limited to this. The second objective optical system may be an optical system configured to objectively acquire information different from the eye refractive power of the subject eye. As an example, the second objective optical system may be an optical system configured to acquire at least one of eye position information of the subject eye, a pupillary distance, and the like.

For example, by attaching the attachment having such an optical system to the eye examination device, it is possible to convert the first objective optical system configured to objectively acquire the eye refractive power of the subject eye or information on the subject eye, into the second objective optical system configured to objectively measure information different from the eye refractive power of the subject eye. Therefore, by using one eye examination device, it is possible to easily acquire the eye refractive power of the subject eye or the information on the subject eye, and the information different from the eye refractive power of the subject eye.

The controller may be configured to perform control based on the detection signal obtained in response to detecting at least one of mounting of the attachment to the eye examination device, and the electrical connection between the eye examination device and the attachment. For example, the controller may be configured to perform automatic switching from the first mode in which image capturing, examination, measurement, and the like are performed by the first objective optical system, to the second mode in which image capturing, examination, measurement, and the like are performed by the second objective optical system, and to output of the leading information for setting change.

The present disclosure is not limited to the device described in the present embodiment. For example, terminal control software (program) that performs the functions of the above embodiments can be supplied to a system or a device via a network or various storage media, and a control device (for example, a CPU and the like) of the system or the device can read and execute the program.

<First Example>

A first example of the eye examination device according to the present embodiment will be described.

<Device Configuration>

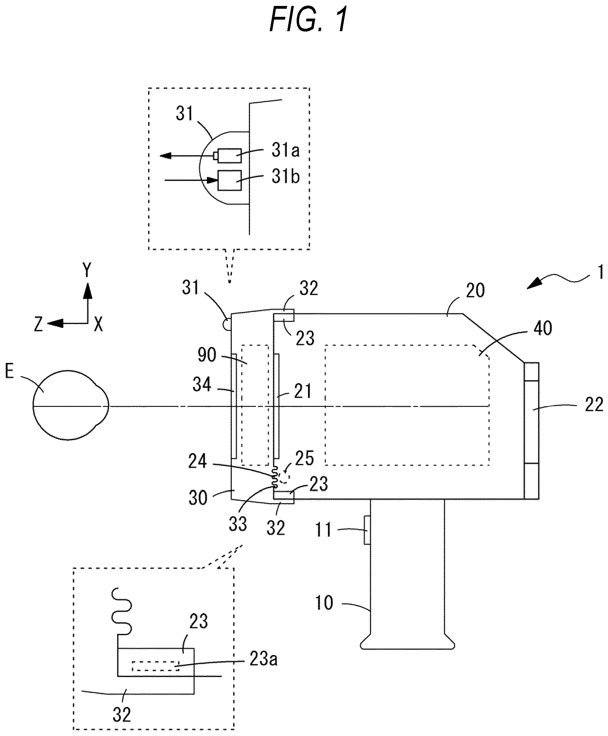

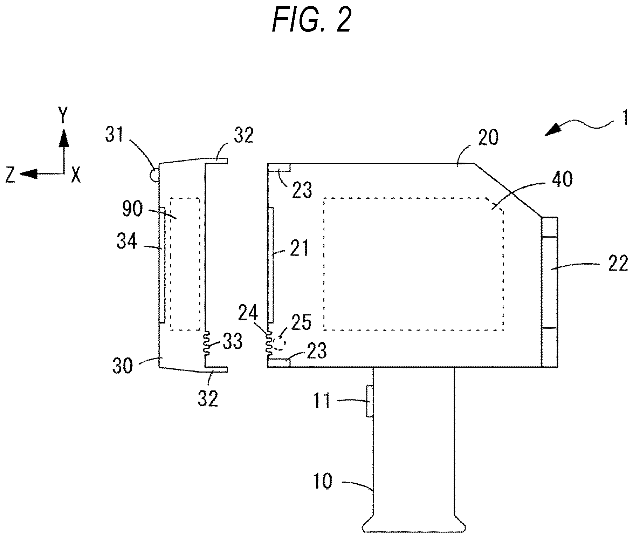

FIGS. 1 and 2 are external views of an eye examination device 1. FIG. 1 illustrates a state in which an attachment is mounted to the eye examination device 1. FIG. 2 illustrates a state in which the attachment is removed from the eye examination device 1. Here, as the eye examination device, a hand-held eye refractive power measurement device will be described as an example.

The eye examination device 1 includes a handle portion 10, a housing portion 20, an attachment portion 30, and the like. The handle portion 10 includes an operation unit 11 and the like. The operation unit 11 is a button through which an operation signal for starting measurement of a subject eye E is input.

The housing portion 20 includes a presentation window 21, a display unit 22, a mounting portion 23, an electrical connection portion 24, and the like. The presentation window 21 is formed by a transparent member (for example, a panel) made of acrylic resin, a glass plate, or the like. The display unit 22 is configured to display an anterior segment observation image of the subject eye E, measurement results, and the like. The display unit 22 may be a touch panel that also functions as the operation unit 11, and may further include buttons through which operation signals related to various settings are input.

The attachment portion 30 includes a distance measurement unit 31, a mounting portion 32, an electrical connection portion 33, a presentation window 34, and the like. The distance measurement unit 31 is an ultrasonic sensor configured to measure the distance from the subject eye E to the attachment portion 30. The ultrasonic sensor includes an ultrasonic transmission unit 31a configured to transmit an ultrasonic wave toward the subject eye E, and an ultrasonic reception unit 31b configured to receive an ultrasonic wave reflected from the subject eye E. The presentation window 34 is formed by a transparent member (for example, a panel) made of acrylic resin, a glass plate, or the like.

In the present embodiment, the attachment portion 30 is detachably mounted to the housing portion 20. For example, the mounting portion 23 and the mounting portion 32 are made of a ferromagnetic material. The mounting portion 23 and the mounting portion 32 are attached by a mutual magnetic force, and are removed by applying a force greater than the magnetic force. The mounting portion 23 and the mounting portion 32 may be attached with a configuration different from the ferromagnetic material (as an example, fitting of a convex portion and a concave portion, and the like). The mounting portion 23 and the mounting portion 32 may be attached with a combination of a ferromagnetic material and a configuration different from the ferromagnetic material.

The mounting portion 23 includes a mounting detector 23a configured to detect a contact between the mounting portion 23 and the mounting portion 32. The mounting detector 23a may be a contact sensor (for example, a microswitch and the like) or a non-contact sensor (for example, an optical sensor, a magnetic sensor, and the like). An output signal from the mounting detector 23a is input to the controller 130. Thus, mounting of the attachment portion 30 to the housing portion 20 is detected.

In addition, in the present embodiment, the attachment portion 30 is mounted to the housing portion 20 to connect the electrical connection portion 33 of the attachment portion 30 to the electrical connection portion 24 of the housing portion 20. For example, the electrical connection portion 33 is configured by a connector, and the electrical connection portion 24 is configured by a jack. The terminal and the terminal hole are configured to come into contact with each other by a mutual connection and to transmit and receive electrical signals. In addition, a connection of the electrical connection portion 33 and the electrical connection portion 24 are not limited to a direct connection, but may be a connection via a USB cable or the like (that is, wired communication) to transmit and receive electrical signals, or may be a connection via Bluetooth (registered trademark) or the like (that is, wireless communication) to transmit and receive electrical signals.

The electrical connection portion 24 includes a connection detector 25 configured to detect an electrical connection between the electrical connection portion 33 and the electrical connection portion 24. The connection detector 25 may be a non-contact sensor (for example, a current sensor, a voltage sensor, and the like). An output signal from the connection detector 25 is input to the controller 130. Thus, the electrical connection between the attachment portion 30 and the housing portion 20 is detected.

A first measurement unit 40 configured to objectively measure the eye refractive power of the subject eye E using the method different from the photorefraction method is stored inside the housing portion 20. Here, a case where a pattern index is projected onto the fundus of the subject eye as the measurement light flux, and the eye refractive power of the subject eye E is objectively measured based on the reflected light flux of the measurement light flux, will be described. A second measurement unit 90 configured to objectively measure the eye refractive power of the subject eye E using the photorefraction method is stored inside the attachment portion 30. For example, the objective measurement using the photorefraction method means that the eye refractive power of the subject eye E is objectively measured from the ratio of the fundus reflected light of the subject eye E in the pupil.

In the present example, by mounting the attachment portion 30 to the housing portion 20, it is possible to convert the measurement of the eye refractive power of the subject eye E, from the method different from the photorefraction method to the photorefraction measurement. That is, the second measurement unit 90 functions as a conversion unit configured to convert the measurement method using the first measurement unit 40.

<First Measurement Unit>

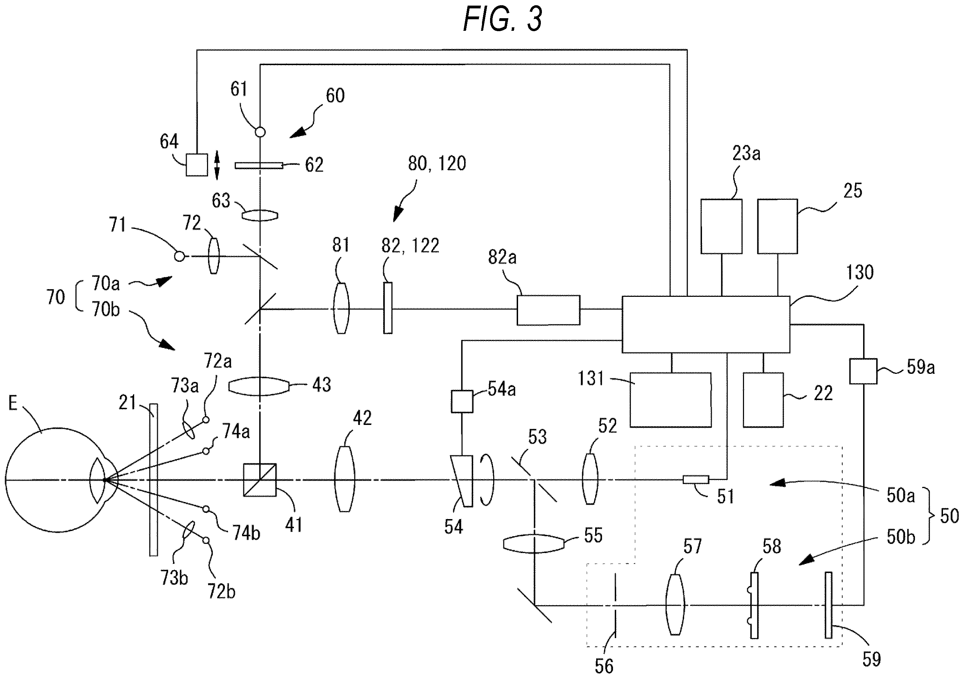

First, the first measurement unit 40 will be described. FIG. 3 is a schematic configuration diagram of the first measurement unit 40. The first measurement unit 40 includes a first measurement optical system 50, a fixation target presentation optical system 60, an index projection optical system 70, an anterior segment observation optical system 80, and the like. The first measurement optical system 50 is configured to objectively measure the eye refractive power (for example, spherical power, columnar power, an astigmatic axis angle, and the like) of the subject eye E using the method different from the photorefraction method. The fixation target presentation optical system 60 is configured to present a fixation target to the subject eye E. The index projection optical system 70 is configured to project an alignment index onto the subject eye E. The anterior segment observation optical system 80 is configured to capture the anterior segment of the subject eye E.

A beam splitter 41 is disposed in front of the subject eye E. The beam splitter 41 is configured to guide the measurement light flux from the fixation target presentation optical system 60 to the subject eye E. Further, the beam splitter 41 is configured to guide the reflected light flux from the anterior segment of the subject eye E to the anterior segment observation optical system 80.

[First Measurement Optical System]

The first measurement optical system 50 includes a light projection optical system 50a and a light reception optical system 50b in a transmission direction of the beam splitter 41. The light projection optical system 50a includes a light source 51, a relay lens 52, a hole mirror 53, a prism 54, an objective lens 42, and the like. The light source 51 is in an optically conjugate positional relationship with the fundus. The aperture of the hole mirror 53 is in an optically conjugate positional relationship with the pupil. The prism 54 is disposed at a position deviated from the optically conjugate position with the pupil, and decenters the light flux passing through the prism 54 with respect to the optical axis. The prism 54 is configured to be rotationally driven about the optical axis by a drive unit 54a. Instead of the prism 54, a plane-parallel plate may be obliquely disposed on the optical axis.

The light reception optical system 50b includes the objective lens 42, the prism 54, the hole mirror 53, the relay lens 55, a light reception diaphragm 56, a collimator lens 57, a ring lens 58, an imaging element 59, and the like. In the light reception optical system 50b, the objective lens 42, the prism 54, and the hole mirror 53 are shared with the light projection optical system 50a. The light reception diaphragm 56 is in an optically conjugate positional relationship with the fundus. The ring lens 58 is in an optically conjugate positional relationship with the pupil. The imaging element 59 is in an optically conjugate positional relationship with the fundus.

In such a configuration of the first measurement optical system 50, the measurement light flux emitted from the light source 51 passes through the relay lens 52, the hole mirror 53, the prism 54, the objective lens 42, and the beam splitter 41, and then is projected onto the fundus, as a spot-shaped light flux, through the presentation window 21. As a result, a point light source image is formed on the fundus. At this time, the prism 54 is rotated around the optical axis, and a pupil projection image (projected light flux on the pupil) at the aperture of the hole mirror 53 is eccentrically rotated at a high speed. The reflected light flux that is the measurement light flux reflected from the fundus is reflected by the hole mirror 53 via the beam splitter 41, the objective lens 42 and the prism 54. The reflected light flux further passes through the relay lens 55 and is condensed at a position of the light reception diaphragm 56, and is formed as a ring-shaped image (ring image) on the imaging element 59 by the collimator lens 57 and the ring lens 58. An output signal from the imaging element 59 is input to the controller 130 via an image processing unit 59a, and the eye refractive power is calculated.

The first measurement optical system 50 projects the measurement light flux from the light source 51 onto either the left eye or the right eye, and extracts the reflected light flux from the fundus of the left eye or the right eye in a ring shape to form an image on the imaging element 59.

Further, the first measurement optical system 50 only needs to be an optical system including the light projection optical system configured to project the measurement light flux onto the fundus of the subject eye E, and the light reception optical system configured to receive the reflected light flux that is the measurement light flux reflected from the fundus. Alternatively, an optical system different from that in the present embodiment may be used. For example, the first measurement optical system 50 may be an optical system configured to project a spot index onto the fundus and to use a Shack-Hartmann sensor to detect the reflected light flux of the spot index on the fundus. Further, for example, the first measurement optical system 50 may be a phase-difference optical system configured to project a slit onto the subject eye E.

[Fixation Target Presentation Optical System]

The fixation target presentation optical system 60 includes a light source 61, a fixation target plate 62, a projection lens 63, an objective lens 43, and the like in a reflection direction of the beam splitter 41. A fixation target is illuminated the fixation target plate 62 with the light source 61 to be presented to the subject eye E. The fixation target plate 62 is used in a case where the subject eye E is fixed and the eye refractive power is measured. The drive unit 64 can fog the subject eye E by moving the light source 61 and the fixation target plate 62 in an optical axis direction. Further, the drive unit 64 can move the fixation target plate 62 in the optical axis direction to move a presentation position of the fixation target with respect to the subject eye E.

[Index Projection Optical System]

The index projection optical system 70 includes an XY index projection optical system 70a and a Z index projection optical system 70b. The XY index projection optical system 70a is configured to project an alignment index for detecting the alignment state of the subject eye E in the left, right, up, and down directions (XY directions). The Z index projection optical system 70b is configured to project an alignment index for detecting the alignment state of the subject eye E in the front and rear direction (Z direction).

The XY index projection optical system 70a includes a light source 71, a condenser lens 72, and the like. The light source 71 is configured to emit near-infrared light. Alignment index light for XY detection, which is emitted from the light source 71, is converted into a parallel light flux (substantially parallel light flux) by the objective lens 43.

The Z index projection optical system 70b includes a first index projection optical system and a second index projection optical system. The first index projection optical system is configured to project an infinite alignment index onto the cornea of the subject eye E. The second index projection optical system is configured to project a finite alignment index onto the cornea of the subject eye E.

The first index projection optical system includes point light sources 72a and 72b, collimator lenses 73a and 73b, and the like. The point light sources 72a and 72b are configured to emit near-infrared light. The collimator lenses 73a and 73b is configured to collimate the light fluxes emitted from the point light sources into parallel light fluxes (substantially parallel light fluxes). For example, a plurality of the point light sources and collimator lenses are disposed on concentric circles with respect to the optical axis at intervals of 45 degrees and are symmetric across a vertical plane passing through the optical axis.

The second index projection optical system includes point light sources 74a and 74b. The point light sources 74a and 74b are configured to emit near-infrared light. For example, a plurality of the point light sources are disposed to have a narrower angle than the point light sources of the first index projection optical system and to be symmetric across a vertical plane passing through the optical axis. The second index projection optical system can also be used as an anterior segment illumination configured to illuminate the anterior segment of the subject eye E, an index for measuring the corneal shape of the subject eye E, and the like.

The Z index projection optical system 70b may be configured to project at least one of a dot index, a ring index (so-called a Mayer ring and the like), a line index, and the like.

[Anterior Chamber Observation Optical System]

The anterior segment observation optical system 80 includes the objective lens 43, an imaging lens 81, an imaging element 82, and the like in the reflection direction of the beam splitter 41. The imaging element 82 is in a positional relationship optically conjugate with the anterior segment of the subject eye E, and configured to receive the reflected light flux from the anterior segment. An output signal from the imaging element 82 is input to the controller 130 and the display unit 22 via an image processing unit 83. The anterior segment observation optical system 80 also serves as an optical system configured to detect an alignment index image formed on the cornea of the subject eye E. The anterior segment observation optical system 80 is configured to detect the position of the alignment index image by the image processing unit 83 and the controller 130.

<Second Measurement Unit>

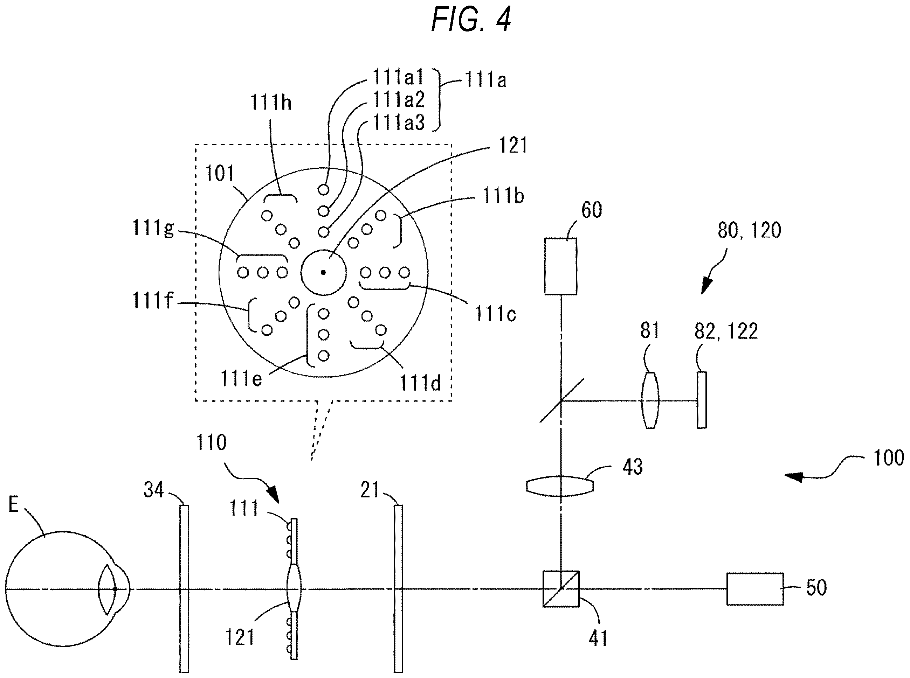

Next, the second measurement unit 90 will be described. FIG. 4 is a schematic configuration diagram of the second measurement unit 90. The second measurement unit 90 includes a second measurement optical system 100. The second measurement optical system 100 is configured to objectively measure the eye refractive power of the subject eye E using the photorefraction method.

[Second Measurement Optical System]

The second measurement optical system 100 includes a light projection optical system 110, a light reception optical system 120, and the like. In the present example, some of optical members configuring the first measurement optical system 50 are shared as the optical members configuring the second measurement optical system 100.

The light projection optical system 110 includes at least a measurement light source 111. The measurement light source 111 is configured to emit near-infrared light. A plurality of measurement light sources 111 are disposed on concentric circles with reference to the optical axis (details will be described later). The light reception optical system 120 includes at least a wide-angle lens 121, an imaging element 122, and the like. The wide-angle lens 121 is configured to widen the image capturing angle-of-view for capturing the anterior segment of the subject eye E. The imaging element 122 is in an optically conjugate relationship with the pupil of the subject eye E.

In the present example, the anterior segment observation optical system 80 also serves as the light reception optical system 120, and the imaging element 82 of the anterior segment observation optical system 80 is used as the imaging element 122 of the light reception optical system 120. That is, the light reception optical system 120 includes the wide-angle lens 121, the objective lens 42, the imaging lens 81, the imaging element 82 (imaging element 122), and the like. Further, by disposing the wide-angle lens 121 in the optical path of the anterior segment observation optical system 80, the image capturing angle-of-view of the anterior segment observation optical system 80 is widened. For example, depending on whether or not the attachment portion 30 is mounted, the image capturing angle-of-view in the first measurement mode described later and the image capturing angle-of-view in the second measurement mode described later are changed.

The measurement light source 111 and the wide-angle lens 121 are provided as an integrated member fixed to a base 101. The measurement light source 111 and the wide-angle lens 121 may be provided as separate members. The measurement light source 111 includes a plurality of measurement light sources, and the respective measurement light sources are disposed to be separated from each other in at least three meridian directions. The measurement light sources can be set in any number of meridian directions (for example, one meridian direction, two meridian directions, four meridian directions, and the like).

In the present example, the case where the measurement light sources 111 are disposed in the four meridian directions will be described. In the present example, the case where the measurement light sources 111 are disposed in order on a virtual straight line extending in the meridian direction with reference to the optical axis center of the second measurement optical system 100. In addition, in one meridian direction, two sets of the measurement light sources 111 are disposed symmetrically with reference to the optical axis center of the wide-angle lens 121.

As the measurement light sources 111, eight sets of measurement light sources (measurement light source 111a, measurement light source 111b, measurement light source 111c, measurement light source 111d, measurement light source 111e, measurement light source 111f, measurement light source 111g, and measurement light source 111h) are disposed in the four meridian directions. For example, the measurement light sources 111a to 111h are concentrically disposed on the outside of the outer circumference of the wide-angle lens 121 at intervals of 45°. Each measurement light source may be disposed at any position.

Each of the measurement light sources 111a to 111h includes three light sources. For example, the three light sources in each measurement light source (as an example, three light sources 111a1, 111a2, and 111a3 in the measurement light source 111a) are disposed, in order at predetermined intervals, in the meridian direction (in other words, radial direction) with reference to the optical axis center of the wide-angle lens 121. Each light source can be independently controlled by the controller 130. For example, the lighting and extinguishing of each light source, the adjustment of the amount of light, and the like can be controlled independently.

In the above description, the configuration in which the measurement light sources 111 are disposed symmetrically with respect to the optical axis center of the second measurement optical system 100 has been described as an example, but the present example is not limited to this. For example, the measurement light sources 111 may be disposed asymmetrically with reference to the optical axis center of the second measurement optical system 100. As an example, the measurement light source 111 may be disposed only on one side of one meridian direction. Further, although the configuration using the eight measurement light sources 111 has been described above, the present example is not limited to this. For example, a configuration using any sets (for example, three sets, four sets, five sets, six sets, and the like) of measurement light sources 111 may be used. In the above description, the measurement light sources 111a to 111h are configured to include three light sources, but the present example is not limited to this. For example, any number of light sources (for example, two, four, five, and the like) may be used.

<Conversion of Measurement Method by Mounting Attachment

In the present example, by mounting the attachment portion 30 to the housing portion 20, the first measurement optical system 50 and the second measurement optical system 100 can be integrated, and the measurement method of the first measurement optical system 50 can be converted. More specifically, objective measurement different from the photorefraction method by the first measurement optical system 50 is converted into objective measurement using the photorefraction method by the first measurement optical system 50 and the second measurement optical system 100.

In the configuration of the first measurement optical system 50 and the second measurement optical system 100 in this case, the fundi of both the left eye and the right eye is irradiated with the measurement light flux emitted from the measurement light source 111. The reflected light flux that is the measurement light flux reflected from the fundus passes through the wide-angle lens 121, is reflected by the beam splitter 41, and is captured by the imaging element 122 (imaging element 82) via the imaging lens 81. At this time, the image capturing angle-of-view of the anterior segment is widened by the disposition of the wide-angle lens 121, and both the reflected light flux from the left eye and the reflected light flux from the right eye are captured by the imaging element 122. An output signal from the imaging element 122 is input to the controller 130 via an image processing unit 82a, and the eye refractive power is calculated.

In a case where the attachment portion 30 is mounted, the index projection optical system 70 (second index projection optical system) of the first measurement optical system 50 is blocked. Therefore, the light projection optical system 105 of the second measurement optical system 100 may be used as an anterior segment illumination. That is, the measurement light source 111 may also have the functions of irradiating the subject eye E with the measurement light flux and illuminating the anterior segment. A dedicated light source configured to illuminate the anterior segment may be provided separately.

<Controller>

The controller 130 includes a CPU (processor), a RAM, a ROM, and the like. The CPU is configured to control driving of each unit in the eye examination device 1. The RAM temporarily is configured to store various types of information. Various programs executed by the CPU are stored in the ROM. The controller 130 may be configured by a plurality of controllers (that is, a plurality of processors).

The controller 130 is electrically connected to the operation unit 11, the display unit 22, the attachment detector 23a, the connection detector 25, the drive unit 54a, the drive unit 64, the image processing unit 59a, the image processing unit 82a, and a non-volatile memory 131 (referred to as a memory 131 below), and the like. In addition, the controller 130 is electrically connected to the ultrasonic transmission unit 31a, the ultrasonic reception unit 31b, the measurement light source 111, and the like via the electrical connection portion 33 and the electrical connection portion 24.

The memory 131 is a non-transitory storage medium configured to retain stored content even in a case where power supply is interrupted. For example, the memory 131 may be a hard disk drive, a flash ROM, a USB memory, or the like. The memory 131 may be configured to store the eye refractive power of the subject eye E and the like.

<Control Action>

A control action of the eye examination device 1 will be described.

The eye examination device 1 can set either of two measurement modes, which are the first measurement mode in which the eye refractive power of the subject eye E is objectively measured using the method different from the photorefraction method, and the second measurement mode in which the eye refractive power of the subject eye E is objectively measured using the photorefraction method. For example, the measurement modes are automatically switched depending on whether the attachment portion 30 is mounted. The examiner may manually switch the measurement mode.

<Measurement by Method Different from Photorefraction Method>

First, a case where the eye refractive power is measured by only the first measurement unit 40 without mounting the attachment portion 30 to the housing portion 20 will be described as an example. The controller 130 is configured to detect that the attachment portion 30 is not mounted, to set the first measurement mode, and to perform control corresponding to the first measurement mode. For example, in the first measurement mode, the eye examination device 1 (housing portion 20 in this case) is brought closer to the subject eye E, and the operation distance from the subject eye E to the eye examination device 1 is shortened. Then, the controller 130 is configured to acquire an anterior segment observation image in which the subject eye E is enlarged (an image of a narrow range including the pupil). In addition, fine alignment is performed in order to measure the eye refractive power more accurately.

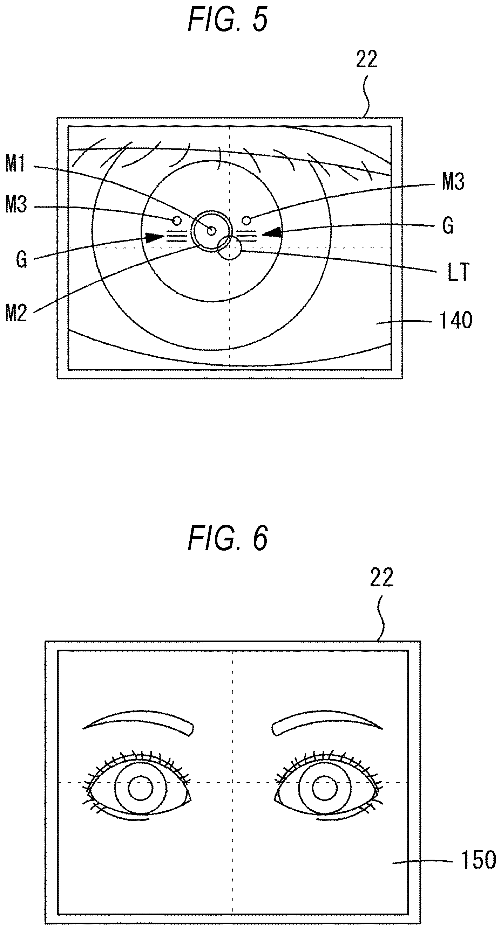

FIG. 5 is an example of the display unit 22 in the first measurement mode. The examiner grips the handle 10 and brings the presentation window 21 closer to the front of the subject eye E. Further, the examiner instructs the subject to fixate the fixation target (fixation target plate 62) through the presentation window 21. Thus, the anterior segment of the subject eye E is captured by the imaging element 82, and an anterior segment observation image 140, an alignment index image M1 by the XY index projection optical system 70a, and an alignment index image (here, a Meyer ring image M2) at a finite distance in the Z index projection optical system 70b, and an infinite alignment index image M3, and the like are displayed on the display unit 22. Further, a reticle LT representing the coordinates of the optical axis of the first measurement unit 40 and the like is displayed on the display unit 22.

The controller 130 is configured to acquire misalignment between the subject eye E and the body (optical axis) of the eye examination device 1 in the XY directions, based on an output signal from the imaging element 82 and an output signal from a detector (not illustrated) configured to detect the two-dimensional position of the alignment index image M1. In addition, based on the output signal from the imaging element 82, the controller 130 is configured to acquire the misalignment between the subject eye E and the body (optical axis) of the eye examination device 1 in the Z direction by utilizing the change in the image interval of the Meyer ring image M2. Further, the controller 130 is configured to increase or decrease the number of indicators G based on the amount of misalignment in the Z direction.

The examiner moves the body of the eye examination device 1 in the XY directions to place the alignment index image M1 in the reticle LT. The body of the eye examination device 1 is moved in the Z direction, and the indicator G is adjusted to a predetermined number (or the Mayer ring image M2 is made narrowest). Thus, the alignment between the subject eye E and the body of the eye examination device 1 is completed. Further, the examiner operates the operation unit 11. As a result, measurement of the subject eye E is started.

Based on the output signal from the operation unit 11, the controller 130 is configured to turn on the light source 51 to irradiate the subject eye E with the measurement light flux, and cause the imaging element 59 to image the reflected light flux from the fundus, to detect the ring image. Further, the controller 130 is configured to analyze the ring image, obtain the eye refractive power in each meridian direction of the ring image, and perform predetermined arithmetic processing on the eye refractive power.

In the present example, in this manner, the eye refractive power of the subject eye E can be objectively measured using the method different from the photorefraction method. For example, the eye refractive power of the left eye and the eye refractive power of the right eye may be obtained by sequentially performing the above control actions for the left eye and the right eye. The eye refractive power (spherical power, cylindrical power, an astigmatic axis angle, and the like) is displayed on the display unit 22 and stored in the memory 131.

< Measurement Using Photorefraction Method>

Next, a case where the attachment portion 30 is mounted to the housing portion 20 and the eye refractive power is measured by using the second measurement unit 90 and the first measurement unit 40 will be described as an example.

The examiner mounts the attachment portion 30 to the housing portion 20. As a result, the mounting portion 32 of the attachment portion 30 and the mounting portion 23 of the housing portion 20 come into contact with each other, and an output signal is emitted from the mounting detector 23a. Also, the electrical connection portion 33 of the attachment portion 30 and the electrical connection portion 24 of the housing portion 20 are electrically connected, and an output signal is emitted from the connection detector 25. The controller 130 is configured to control each member of the attachment portion 30, in addition to each member of the housing portion 20, using the electrical connections.

The controller 130 is configured to automatically switch the measurement mode, based on an output signal from the mounting detector 23a and an output signal from the connection detector 25, from the first measurement mode in which the eye refractive power of the subject eye E is objectively measured using the method different from the photorefraction method, to the second measurement mode in which the eye refractive power of the subject eye E is objectively measured using the photorefraction method. The controller 130 may be configured to switch the measurement mode based on the output signal from the attachment detector 23a or may be configured to switch the measurement mode based on the output signal from the connection detector 25.

The controller 130 is configured to perform control corresponding to the second measurement mode. For example, in the second measurement mode, the eye examination device 1 (here, the attachment portion 30) is separated from the subject eye E to increase the operation distance from the subject eye E to the eye examination device 1, and an anterior segment observation image (image in a wide range including the pupil) in which the subject eye E is reduced is acquired. A rough alignment is also performed to more easily measure the eye refractive power.

FIG. 6 is an example of the display unit 22 in the second measurement mode. The examiner grips the handle 10 and brings the presentation window 34 closer to the front of the subject eye E. The examiner also instructs the examinee to fixate on a fixation lamp through the presentation window 34. For example, at least one light source included in the measurement light source 111 may be turned on as the fixation lamp (bright spot). As a result, the anterior segment of the subject eye E is captured by the imaging element 122, and the anterior segment observation image 150 and the like are displayed on the display unit 22.

The examiner moves the body of the eye examination device 1 in the X, Y, and Z directions such that the left eye and the right eye are within the image capturing angle-of-view. Thus, the alignment between the subject eye E and the body of the eye examination device 1 is completed. In a case where the controller 130 analyzes the anterior segment observation image 150 and detects both the left eye and the right eye, the controller 130 may be configured to generate a sound or display a message to perform a notification indicating that the alignment has been completed. Further, the examiner operates the operation unit 11. As a result, measurement of the subject eye E is started.

The controller 130 is configured to acquire the distance from the subject eye E to the attachment portion 30 using the distance measurement unit 31, based on the output signal from the operation unit 11. For example, the controller is configured to measure the distance from the subject eye E to the front of the attachment portion 30 and add the distance from the attachment portion 30 to the wide-angle lens 121 (a known design value) thereto, to acquire a separation distance (measurement distance) between the subject eye E and the wide-angle lens 121.

Further, based on the output signal from the operation unit 11, the controller 130 is configured to turn on the measurement light source 111 to irradiate the subject eye E with the measurement light flux, and to cause the imaging element 122 to capture the reflected light flux from the fundus.

In the present example, the light sources are turned on in order for each set of measurement light sources. For example, the controller 130 is configured to turn on a light source 111a1 among the measurement light sources 111a. The other measurement light sources 111b to 111h, and light sources 111a2 and 111a3 are turned off. An output signal from the imaging element 122 in a case where the light source 111a1 is turned on is stored in the memory 131 as a measurement image. In a case where a measurement image by turning on the light source 111a1 is acquired, the controller 130 is configured to turn off the light source 111a1, and to turn on the next light source 111a2, and thus similarly acquires the measurement image. Further, in a case where the measurement image obtained by turning on the light source 111a2 is acquired, the controller 130 is configured to turn off the light source 111a2, and to turn on the next light source 111a3, and thus similarly acquires the measurement image.

In a case where measurement images using the three light sources in one set of measurement light sources 111a are acquired, the controller 130 is configured to sequentially perform measurements with the remaining sets of measurement light sources 111b to 111h. The order in which the measurement light sources of each set are turned on and the order in which the three light sources included in each set of measurement light sources are turned on are not limited to this, and may be any order.

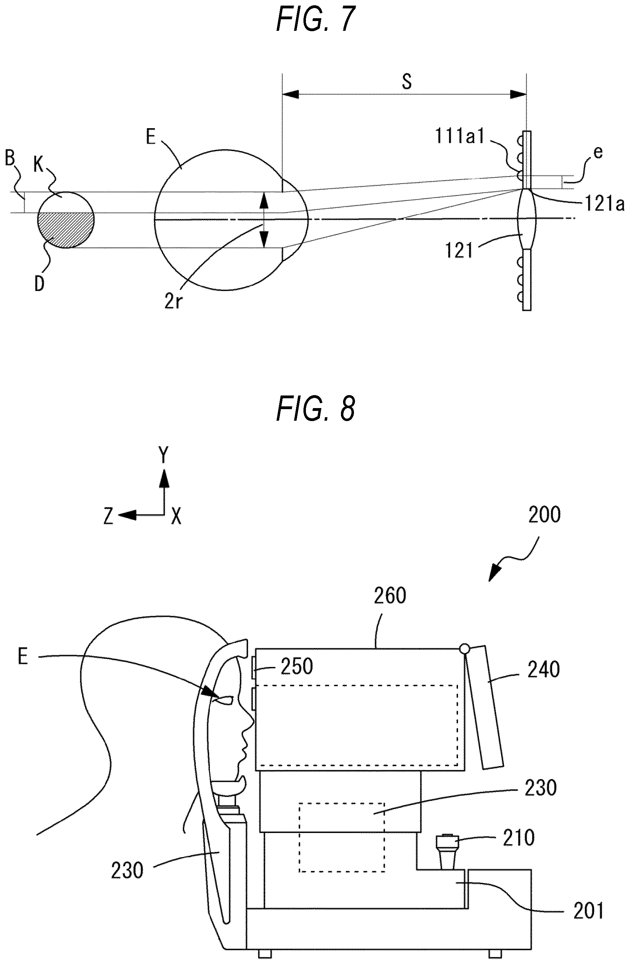

FIG. 7 is a diagram illustrating the photorefraction method. The controller 130 is configured to analyze each measurement image to obtain the eye refractive power of the subject eye E. More specifically, the controller 130 is configured to detect the ratio of the radial dimension of the bright crescent in the pupil of the anterior segment included in each measurement image to the pupil diameter, and to acquire the eye refractive power by the following expression (see, for example, JP2006-149501A).

$$R = 1 - \{eL/2r(A + L)\}$$

Here, R denotes the dimension ratio (B/2r) of the bright crescent K in the pupil to the pupil diameter. B indicates the length of the bright crescent K in the radial direction of the pupil. r indicates the radius of the pupil of the subject eye E. A indicates the eye refractive power of the subject eye E. e indicates the distance from the end 121a of the wide-angle lens 121 to the measurement light source 111 (the light source 111a1 in the measurement light source 111a is illustrated in FIG. 7). L is the reciprocal of the distance S between the subject eye E and the wide-angle lens 121 (L = 1/S).

For example, the ratio R of the bright crescent K varies depending on the eye refractive power A of the subject eye, provided that other conditions are constant. That is, the eye refractive power A of the subject eye E is calculated from the ratio R of the bright crescents measured under a predetermined condition, using the following expression.

$$A = \{eL/2r(1 - R)\} - L$$

The controller 130 is configured to calculate spherical information (spherical power) in the meridian direction in which the measurement light source 111a is disposed, based on the measurement image obtained by lighting the measurement light source 111a. For example, at this time, the controller 130 is configured to acquire the spherical information based on at least one of the measurement images obtained by turning on the three light sources 111a1 to 111a3. In this case, the average value of the spherical information for each light source may be acquired as the spherical information of the measurement light source 111a in the meridian direction. Alternatively, one piece of spherical information for each light source may be selected and acquired as spherical information of the measurement light source 111a in the meridian direction.

The controller 130 is configured to similarly calculate spherical information in the meridian direction for the other measurement light sources 111b to 111h. In a case where the controller 130 acquire the spherical information in all the meridian directions, the controller 130 is configured to acquire the spherical power of the subject eye E based on the spherical information. Further, the controller 130 is configured to acquire the cylindrical power and the astigmatic axis angle of the subject eye E, based on the spherical power in the meridian direction (spherical power distribution).

In the present example, in this manner, the eye refractive power of the subject eye E can be objectively measured using the photorefraction method. For example, in the above control action, both the left eye and the right eye are included in the measurement image. Thus, it is possible to acquire the eye refractive power of the left eye and the eye refractive power of the right eye by performing analysis for the left eye and the right eye. The eye refractive power is displayed on the display unit 22 and stored in the memory 131.

<Second Example>

A second example of the eye examination device according to the present embodiment will be described. In the first example and the second example, the same component is denoted by the same reference sign, and the description thereof will not be repeated.

<Device Configuration>

FIG. 8 is an external view of an eye examination device 200. Here, as the eye examination device, a stationary eye refractive power measurement device will be described as an example. In the second example, the second measurement optical system 100 is incorporated in the housing as a portion of the first measurement optical system 50.

The eye examination device 200 includes a moving table 201, an operation unit 210, a drive unit 220, a face support portion 230, a display unit 240, a distance measurement unit 250, a measurement unit 260, and the like. The operation unit 210 includes a button through which an operation signal for starting measurement of a subject eye E is input. The operation unit 210 includes a lever through which the operation signal for moving the measurement unit 260 with respect to the subject eye E is input. The drive unit 220 configured to move the measurement unit 260 in the X direction, the Y direction, and the Z direction with respect to the moving table 201. The face support portion 230 is configured to support the face of the examinee. For example, the face support portion 230 may include at least one of a forehead rest and a chin rest. The display unit 240 is configured to display an anterior segment observation image of the subject eye E, measurement results, and the like. The display unit 240 may be a touch panel that also functions as the operation unit 210. The distance measurement unit 250 is an ultrasonic sensor configured to measure the distance from the subject eye E to the measurement unit 260. The measurement unit 260 is configured to objectively measure the eye refractive power of the subject eye E using the photorefraction method or the method different from the photorefraction method.

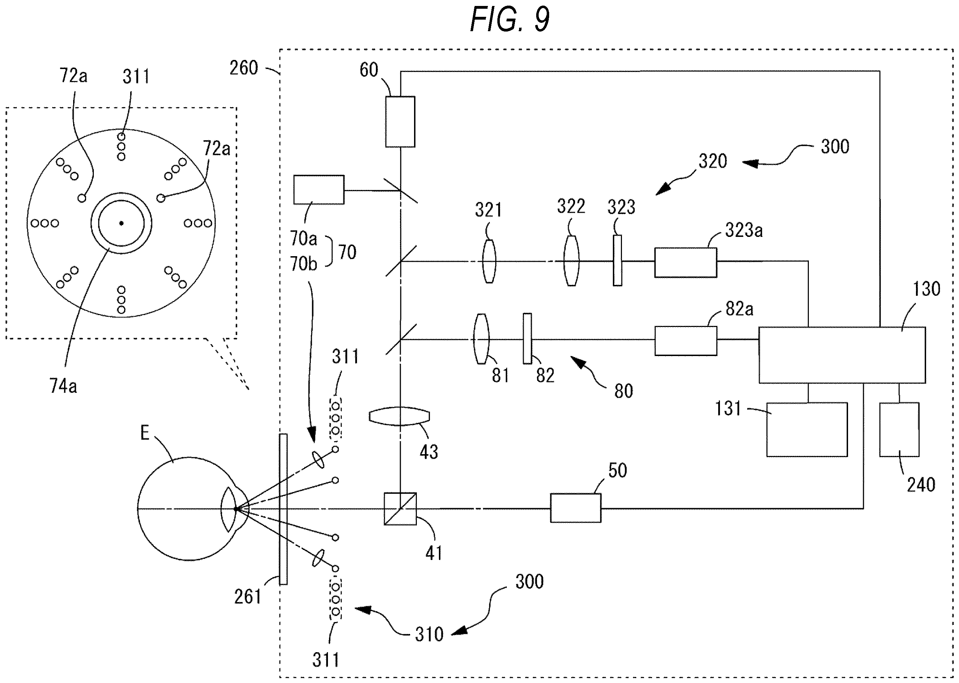

FIG. 9 is a schematic configuration diagram of the measurement unit 260. The measurement unit 260 includes the first measurement optical system 50, a second measurement optical system 300, the fixation target presentation optical system 60, the index projection optical system 70, the anterior segment observation optical system 80, and the like.

The second measurement optical system 300 is configured to objectively measure the eye refractive power of the subject eye E using the photorefraction method. The second measurement optical system 300 includes a light projection optical system 310, a light reception optical system 320, and the like. The light projection optical system 310 includes at least a measurement light source 311. As with the measurement light source 111 described above, the measurement light source 311 includes eight sets of measurement light sources disposed in the four meridian directions, and each set of measurement light sources includes three light sources. The second measurement optical system 300 includes the objective lens 43, a wide-angle lens 321, an imaging lens 322, an imaging element 323, and the like in the reflection direction of the beam splitter 41. The wide-angle lens 321 is configured to widen the image capturing angle-of-view of the imaging element 323. The imaging element 323 is in a positional relationship optically conjugate with the pupil of the subject eye E, and is configured to receive the reflected light flux from the anterior segment.

In the configuration of the second measurement optical system 300 in this case, the measurement light flux from the measurement light source 311 is reflected by the fundus, subsequently reflected by the beam splitter 41, passes through the wide-angle lens 321 and the imaging lens 322, and captured by the imaging element 323. An output signal from the imaging element 323 is input to the controller 130 and the display unit 240 via an image processing unit 323a. In the present example, the image capturing angle-of-view of the imaging element 323 is set to be wider than the image capturing angle-of-view of the imaging element 82 by disposing the wide-angle lens 321. For example, the imaging element 82 has an image capturing angle-of-view capable of capturing an image of the one subject eye E, and the imaging element 323 has an image capturing angle-of-view capable of capturing an image of both subject eyes E.

In the present example, the light reception optical system 320 is disposed on the back side (the side away from the subject eye) from the objective lens 43, but the present example is not limited to this. For example, the light reception optical system 320 may be disposed on the front side (the side closer to the subject eye) of the objective lens 43. In this case, the optical path may be branched by a half mirror or the like between the beam splitter 41 and the objective lens 42, and the wide-angle lens 321, the imaging lens 322, the imaging element 323, and the like may be disposed.

<Control Action>

A control action of the eye examination device 200 will be described.

The eye examination device 200 also is configured to set either of two measurement modes, which are the first measurement mode in which the eye refractive power of the subject eye E is objectively measured using the method different from the photorefraction method, and the second measurement mode in which the eye refractive power of the subject eye E is objectively measured using the photorefraction method. For example, the measurement modes are switched based on the operation of the examiner on the operation unit 210 (or the display unit 240).

<Measurement Using Method Different from Photorefraction Method>

The examiner operates the operation unit 210 to operate a button (not illustrated) for selecting the measurement mode for the subject eye E. The controller 130 is configured to set the first measurement mode based on the output signal from operation unit 210, and to perform control corresponding to the first measurement mode.

The examiner instructs the examinee to fixate the fixation target (fixation target plate 62) with the face abutting on the face support portion 230. As a result, the anterior segment of the subject eye E and each alignment index image are captured by the imaging element 82. The controller 130 is configured to detect the corneal vertex position using each alignment index image, to drive the drive unit 220 based on the deviation of the corneal vertex position with respect to the alignment reference position in the X direction, the Y direction, and the Z direction, to complete auto-alignment between the subject eye E and the body of the eye examination device 200.

Further, the examiner operates the operation unit 210 to start measurement of the subject eye E. Based on the output signal from the operation unit 210, the controller 130 is configured to control the light source 51, the imaging element 59, and the like, and to analyze the ring image to measure the eye refractive power of the subject eye E, as in the first example.

< Measurement Using Photorefraction Method>

The examiner operates the operation unit 210 to operate a button (not illustrated) for selecting the measurement mode for the subject eye E. The controller 130 is configured to set the second measurement mode based on the output signal from operation unit 210, and to perform control corresponding to the second measurement mode.

In the second example, the wide-angle lens 321 is not disposed on the optical axis of the fixation target presentation optical system 60. Thus, even in the photorefraction measurement of the subject eye E, the fixation target (fixation target plate 62) can be used to guide the fixation of the subject eye E. At least one light source in the measurement light source 311 may be configured to turn on as a fixation lamp (bright spot). The anterior segment of the subject eye E is captured by the imaging element 323 and displayed on the display unit 240 as an anterior segment observation image.

The controller 130 is configured to detect position information (for example, coordinates) of the left eye and the right eye using the luminance of the anterior segment observation image, and to drive the drive unit 220 based on the position information to place the left eye and the right eye within the image capturing angle-of-view of the imaging element 323. Thus, the auto-alignment between the subject eye E and the body of the eye examination device 200 is completed. Further, the controller 130 is configured to acquire the distance from the subject eye E to the measurement unit 260 using the distance measurement unit 250. The controller 130 is configured to measure the distance from the subject eye E to the front of the measurement unit 260 and to add the distance from the measurement unit 260 to the wide-angle lens 321 (a known design value) thereto, to acquire the distance (measurement distance) between the subject eye E and the wide-angle lens 321.

The examiner operates the operation unit 210 to start measurement of the subject eye E. Based on the output signal from the operation unit 210, the controller 130 is configured to sequentially turn on the measurement light sources 311 and to capture each measurement image with the imaging element 323. Further, the controller 130 is configured to measure the eye refractive power of the subject eye E based on the ratio of the crescent K formed in the pupil of the subject eye E.

The eye examination device 1 and the eye examination device 200 described above can be used to perform at least one of measurement in the first measurement mode using the method different from the photorefraction method and measurement in the second measurement mode using the photorefraction method. In acquiring the eye refractive power of the subject eye E, in the first measurement mode, more accurate alignment of the body (the housing portion 20 or the measurement unit 260) of the eye examination device 1 with respect to the subject eye is required. On the other hand, in the second measurement mode, strict alignment of the body (the attachment portion 30 or the measurement unit 260) of the eye examination device 1 with respect to the subject eye E is not required, and at least an image of the anterior segment of the subject eye E only needs to be captured. That is, the allowable range of alignment of the subject eye E differs between the first measurement mode and the second measurement mode. For this reason, for example, in a case where it is not possible to perform the alignment well when the first measurement mode is applied, or in a case where it is not possible to satisfactorily obtain the measurement result of the subject eye E, the simple measurement results are efficiently acquired by performing switching to application of the second measurement mode.

As described above, the attachment in the present example includes the conversion optical system that is mounted to the eye examination device including the first objective optical system that objectively acquires the information on the subject eye. The conversion optical system is configured to convert the first objective optical system into the second objective optical system configured to objectively measure the eye refractive power of the subject eye using the photorefraction method. As a result, it is possible to easily acquire, using one eye examination device, the information on the subject eye and the eye refractive power of the subject eye using the photorefraction method. In addition, it is possible to handle the image capturing, the measurement, the examination, and the like in accordance with the various situations of the subject eye.

The conversion optical system included in the attachment in the present example includes at least one of a plurality of measurement light sources disposed in the meridian direction with reference to the optical axis center of the conversion optical system, and a wide-angle lens configured to widen the image capturing angle-of-view of the objective optical system of the eye examination device as compared with when the attachment is not mounted. As a result, it is possible to easily convert the objective optical system into the photorefraction optical system by mounting an attachment, and it is possible to handle various situations of the subject eye.

The attachment in the present example includes a distance measurement unit configured to measure the distance from the subject eye to the eye examination device. In a case where the eye refractive power of the subject eye is measured using the photorefraction method, it is necessary to obtain the measurement distance (operation distance) of the subject eye, and it is possible to appropriately acquire the eye refractive power by providing the distance measurement unit. Although it is possible to use the distance measurement unit provided in the eye examination device, it may be difficult to obtain results depending on the configuration of the distance measurement unit in a case where the attachment is mounted. Therefore, by providing the distance measurement unit in the attachment, the measured distance can be obtained more reliably.

In addition, in the attachment in the present example, the distance measurement unit includes the ultrasonic transmission unit configured to transmit an ultrasonic wave toward the subject eye, and an ultrasonic reception unit configured to receive an ultrasonic wave reflected from the subject eye. In the photorefraction measurement with respect to the subject eye, the image capturing angle-of-view of the objective optical system is widened. For this reason, for example, in a configuration in which the distance is measured based on an alignment index image or a bright spot image projected onto the subject eye, the images may be captured small together with the subject eye, and it may be difficult to detect the alignment index image or the bright spot image. On the other hand, in a case where the configuration using ultrasonic waves is provided, transmission and reception of ultrasonic waves do not influence changes in the image capturing angle-of-view of the objective optical system. Thus, it is easy to measure the distance.

Further, the eye examination device in the present example includes the mounting portion to which the attachment is to be mounted, and the connection portion configured to electrically connect the eye examination device and the attachment. In addition, the attachment is used in a case where the connection portion electrically connects the eye examination device and the attachment. Therefore, in the photorefraction measurement of the subject eye, it is possible to easily control the irradiation with the measurement light flux from the light source, image capturing of the reflected light flux from the fundus of the subject eye, and the like. As an example, it is possible to easily synchronize lighting of each light source and image capturing by the detector.

Further, the eye examination device in the present example is configured to detect at least one of mounting of the attachment to the eye examination device and the electrical connection between the eye examination device and the attachment, and to control the action of the eye examination device based on the detection signal. Depending on whether the attachment is attached, it is possible to efficiently measure the eye refractive power of the subject eye by any one measurement method.

Further, the eye examination device in the present example is configured to automatically switch the mode from the first mode using the first objective optical system to the second mode using the second objective optical system, based on the detection signal obtained in response to detection of mounting of the attachment and the electrical connection of the attachment. As a result, it is possible to smoothly start the photorefraction measurement of the examiner simply by mounting the attachment.

Further, in the eye examination device in the present example, the first objective optical system is configured to objectively measure the eye refractive power of the subject eye using the method different from the photorefraction method. That is, the eye examination device is configured to measure the eye refractive power of the subject eye using the method different from the photorefraction method, and furthermore, the eye examination device is configured to measure, by mounting the attachment, the eye refractive power of the subject eye using the photorefraction method. Therefore, for example, the mode can be appropriately used in accordance with the age of the examinee and the quality of the measurement results obtained by each measurement method.

The eye examination device in the present example is configured to control at least one of the first objective measurement unit configured to objectively measure the eye refractive power of the subject eye using the method different from the photorefraction method, and the second objective measurement unit configured to objectively measure the eye refractive power of the subject eye using the photorefraction method, and to acquire the eye refractive power. Thus, for example, it is possible to acquire the eye refractive power of the subject eye in accordance with various situations. As an example, it is possible to acquire the eye refractive power of the subject eye by using the objective measurement unit suitable for the purpose, the application, the measurement accuracy, and the like.

Further, in the eye examination device in the present example, the first objective measurement unit measures the eye refractive power by projecting the measurement light flux onto the fundus of one of the subject eyes, and the second objective measurement unit measures the eye refractive power by projecting the measurement light fluxes onto the fundi of both of the subject eyes. Therefore, for example, in the first objective measurement unit, more precise alignment than the second objective measurement unit is required, but it is possible to improve the measurement accuracy. Further, for example, although the second objective measurement unit has lower measurement accuracy than the first objective measurement unit, it is possible to obtain the measurement results simply and efficiently. The second objective measurement unit may be used for screening for refractive error of the subject eye.

Further, in the eye examination device in the present example, in the first objective measurement unit, the first detector configured to receive the reflected light flux that is the measurement light flux reflected from the fundus of the subject eye is disposed at the fundus conjugate position of the subject eye. Further, in the second objective measurement unit, a second detector configured to receive a reflected light flux of the measurement light flux reflected from the fundus of the subject eye is disposed at a pupil conjugate position of the subject eye. That is, the first objective measurement unit is a fundus conjugate system, and the second objective measurement unit is a pupil conjugate system. Thus, for example, when measuring the eye refractive power of an eye to be subject eye, two measurement methods can be used according to the situation.

Further, in the eye examination device in the present example, the first objective measurement unit is configured to project the pattern index, as a measurement light flux, onto the fundus of the subject eye, and the reflected light flux that is the measurement light flux reflected from the fundus is received by the first detector, to acquire the eye refractive power based on the reflected light flux received by the first detector. For example, the reflected light flux may be extracted as a ring image, and the eye refractive power may be obtained based on the ring image. As a result, for example, by using the first objective measurement unit, it is possible to obtain more information on the meridian direction than by using the second objective measurement unit (photorefraction method). Thus, the eye refractive power of the subject eye is measured with high accuracy.

Further, the eye examination device in the present example includes first alignment unit configured to capture an image of the subject eye and to adjust the positional relationship between the subject eye and the first objective measurement unit, and a second alignment unit configured to capture an image of the subject eye at a wider angle than an angle in the first alignment unit and to adjust the positional relationship between the subject eye and the second objective measurement unit. Therefore, in a case where the eye refractive power of the subject eye is measured, it is possible to perform appropriate alignment in each method. For example, in the measurement method different from the photorefraction method, since an image of a narrow range of the subject eye is captured, more precise alignment may be performed. In the photorefraction method, since an image of a wide range of the subject eye is captured, rough alignment may be performed.

Further, the eye examination device in the present example is configured to set one of a first measurement mode using the first objective measurement unit and a second objective measurement mode using the second objective measurement unit, based on a measurement mode switching signal for measuring the eye refractive power of the subject eye. This allows the examiner to smoothly set the measurement mode and easily start measurements using different measurement methods.

Further, in the eye examination device in the present example, the first objective measurement unit includes an anterior segment capturing optical system configured to capture an image of the anterior segment. In addition, the detector of the second objective measurement unit is also used as the detector included in the anterior segment capturing optical system. This makes it possible to measure the eye refractive power using the method different from the photorefraction method or using the photorefraction method with a simple configuration, without providing a dedicated detector for each optical system.

<Modification>

In the first example, the configuration in which the attachment portion 30 is attached to and detached from the hand-held eye examination device 1 has been described as an example, but the present example is not limited to this. The attachment portion 30 may be detachably attached to the stationary eye examination device. For example, in a case where a stationary eye examination device is provided with an optical system configured to objectively measure the eye refractive power of the subject eye E using the method different from the photorefraction method, it is possible to convert the optical system to the photorefraction measurement by mounting the attachment portion 30.

In the first example, the configuration in which the attachment portion 30 includes the measurement light source 111 and the wide-angle lens 121 has been described as an example, but the present example is not limited to this. For example, the attachment portion 30 may include only the measurement light source 111, and the wide-angle lens 121 may be provided inside the housing portion 20. Further, for example, the attachment portion 30 may be configured to include only the wide-angle lens 121, and the measurement light source 111 may be configured to be provided in the housing portion 20.

In a case where the attachment portion 30 includes only the measurement light source 111, the wide-angle lens 121 may be provided on the optical path from the presentation window 21 to the imaging element 122 to be inserted or removed. For example, the wide-angle lens may be provided near the objective lens 43, near the imaging lens 81, or the like. The controller 130 may be configured to insert and remove the wide-angle lens 121 in conjunction with attachment/detachment of the attachment portion 30, the electrical connection of the attachment portion 30, selection of the first measurement mode or the second measurement mode by the examiner, and the like, and may be configured to switch the image capturing angle-of-view of the subject eye E according to each measurement mode.

Further, in addition to the wide-angle lens 121, an imaging element different from the imaging element 122 may be provided, the optical path may be branched, and the optical members may be fixedly disposed. As an example, the optical path may be branched at any position between the objective lens 43 and the imaging lens 81. The controller 130 may be configured to switch the imaging element to be used in conjunction with each measurement mode.

In a case where the attachment portion 30 includes only the wide-angle lens 121, the light source of the index projection optical system 70 may also be used as the measurement light source 111 (details will be described later). The measurement light source 111 may be disposed separately from the light source of the index projection optical system 70. The controller 130 may be configured to switch the light source to be used in conjunction with each measurement mode.

In the second example, the stationary eye examination device 200 includes the first measurement optical system 50 and the second measurement optical system 300. However, the present example is not limited to this. The hand-held eye examination device may be configured to include the first measurement optical system 50 and the second measurement optical system 300. In other words, in a hand-held eye examination device, an optical system configured to objectively measure the eye refractive power of the subject eye E using a method different from the photorefraction method and an optical system configured to objectively measure the eye refractive power of the subject eye E using the photorefraction method may be provided together.

In the second example, the configuration in which the measurement unit 260 includes the measurement light source 311 of the light projection optical system 310, and the wide-angle lens 321 and the imaging element 323 of the light reception optical system 320 has been described as an example, but the present example is not limited to this. For example, the light source of the index projection optical system 70 may also be used as the measurement light source 311 (details will be described later). Further, for example, a wide-angle lens 321 may be detachably provided in the optical path from the presentation window 261 to the imaging element 82, and the imaging element 82 included in the anterior segment observation optical system 80 may also be used as the imaging element 323. The measurement light source 311 and the wide-angle lens 321 may be provided as an integrated member, and this member may be inserted and removed in conjunction with each measurement mode.

In the first example, in a case where the imaging element 82 of the anterior segment observation optical system 80 is also used as the imaging element 122 of the light reception optical system 120, the setting of the imaging element may be changed according to each measurement mode. Similarly, in the second example, in a case where the imaging element 82 of the anterior segment observation optical system 80 is also used as the imaging element 323 of the light reception optical system 320, the setting of the imaging element may be changed according to each measurement mode. For example, at least one of the exposure time, gain, and the like of the imaging element may be adjusted to an appropriate value.

In the first example, in a case where the light source of the index projection optical system 70 is also used as the measurement light source 111 of the light projection optical system 110, the setting of the light source may be changed according to each measurement mode. Similarly, in the second example, in a case where the light source of the index projection optical system 70 is also used as the measurement light source 311 of the light projection optical system 310, the setting of the light source may be changed according to each measurement mode. For example, the amount of light and the like emitted from the light source may be adjusted to an appropriate value.

In the first example, the configuration in which the measurement light source 111 is used as a fixation lamp has been described as an example, but the present example is not limited to this. For example, a light source different from the measurement light source 111 may be separately provided as a fixation lamp and turned on to form a bright spot. In this case, the light source may be provided on the optical axis or may be provided around the optical axis. Further, in this case, the measurement light source 111 or a light source different from the measurement light source 111 may blink to attract the attention of the subject eye E.

Since the wide-angle lens 121 is disposed on the optical axis of the fixation target presentation optical system 60 by mounting the attachment portion 30, the operation distance of the subject eye E becomes longer in the second measurement mode than in the first measurement mode, and thus the fixation target plate 62 cannot be visually recognized. Therefore, as described above, fixation of the subject eye E can be guided by using the measurement light source 111 also as the fixation lamp or by preparing a new fixation lamp. An optical member (such as a lens) may be inserted on the optical axis of the fixation target presentation optical system 60 such that the fixation target plate 62 can be visually recognized.

Further, in the second example, it may not be possible to visually recognize the fixation target plate 62 depending on the position at which the wide-angle lens 321 is inserted/removed or fixed. Therefore, the measurement light source 311 may also be used as the fixation lamp, or a separate light source for the fixation lamp may be provided. The fixation lamp may blink.

In the first example, the configuration of automatically setting one of the two measurement modes, the first measurement mode and the second measurement mode, according to attachment/detachment of the attachment portion 30 has been described as an example. The present example is not limited to this. For example, leading information may be output to lead the examiner to change a mode from the first measurement mode to the second measurement mode (or from the second measurement mode to the first measurement mode) according to attachment or detachment of the attachment portion 30. As an example, the leading information may be a voice guide for instructing the examiner to perform the next operation, a message display for urging the examiner to change settings, or the like. As a result, the examiner can set the photorefraction measurement without hesitation after mounting the attachment, and can start the measurement smoothly.

In the first example and the second example, the configuration of acquiring the distance from the subject eye E to the eye examination device by transmission and reception of ultrasonic waves by the distance measurement unit 31 or the distance measurement unit 250 has been described as an example. The present example is not limited to this. For example, a distance measurement member may be configured to be disposed in front of the face of the subject, and an image of the distance measurement member may be configured to be captured together with the anterior segment of the subject eye E to acquire the distance from the subject eye E to the eye examination device. In this case, the distance measurement member may have known dimensions, and it is possible to use a figure or scale of a predetermined size. Based on the actual length of the member for distance measurement, the length on the anterior segment observation image, and the change in imaging magnification due to the placement of the wide-angle lens 121, the controller 130 may be configured to estimate the distance from the subject eye E to the eye examination device. In a case where the pupillary distance of the subject eye E is obtained in advance, it is possible to estimate the distance from the subject eye E to the eye examination device based on the actual pupillary distance of the subject E, the pupillary distance on the anterior segment observation image, and changes in the image capturing associated with the disposition of the wide-angle lens 121.

In the first example and the second example, an example in which the index projection optical system 70 is configured to project a Mayer ring image, and the light source included in the index projection optical system 70 and the measurement light source included in the light projection optical system 110 or the light projection optical system 310 are configured to turn on in different manner according to the first measurement mode and the second measurement mode has been described, but the present example is not limited to this. For example, the index projection optical system 70 may be configured to project a point-like (or line-like) target image instead of the Meyer ring image.

In the alignment of the eye examination device with respect to the subject eye E, in a case where the image ratio between the alignment index image at infinity and the alignment index at finite distance can be detected, the alignment state in the Z direction can be determined even if the Meyer ring image is not used. In the measurement of the subject eye E using the photorefraction method, a plurality of measurement light sources are disposed in the meridian direction, measurement images synchronized with lighting of each light source are acquired, and the eye refractive power is calculated. Therefore, in a case where the index projection optical system 70 is configured to project a dot-like target image and perform alignment, it is also possible to use the index projection optical system 70 to irradiate the measurement light flux from each meridian direction, the light source of the index projection optical system 70 can also be used as the measurement light source of the light projection optical system 110 or the light projection optical system 310. In other words, the index projection optical system 70 may have the roles of both projection of the alignment index onto the subject eye E and irradiation of the measurement light flux in the measurement of the photorefraction method.

As described above, in the eye examination device, the light source of the light projection optical system configured to project light toward the anterior segment (here, the index projection optical system 70 that also serves as illumination of the anterior segment) is also used as the measurement light source of the measurement optical system using the photorefraction measurement method (here, light projection optical system 110 or light projection optical system 310). This makes it possible to measure eye refractive power using a method different from the photorefraction method or using a photorefraction method with a simple configuration without providing a dedicated light source for each optical system.

In the first example and the second example, a patterned target light flux is projected onto the fundus of the subject eye E, and the reflected light flux that is the measurement light flux reflected from the fundus is taken out as a ring image. Although the configuration for measuring the eye refractive power using a method different from a photorefraction method has been described as an example, the present embodiment is not limited to this. For example, a configuration in which scanning is performed on the fundus of the subject eye with a measurement light flux, the reflected light flux of the measurement light flux reflected by the fundus is detected, and the eye refractive power is measured using the method different from the photorefraction method based on the phase different signal may be made. For example, it is also possible to obtain more information in the meridian direction than the photorefraction method by this method. Thus, the eye refractive power of the subject eye can be measured with high accuracy.

In the first example and the second example, the configuration in which the eye refractive power of the subject eye E is acquired in photorefraction measurement (measurement in the second measurement mode) by the second measurement optical system 100 or the second measurement optical system 300 has been described as an example, but the present example is not limited to this. The second measurement optical system may be an optical system configured to acquire data different from the eye refractive power of the subject eye, and may be configured to acquire such data in the measurement in the second measurement mode. For example, in the second measurement mode, both the left eye and the right eye are included in the measurement image, and thus, the pupillary distance of the left eye and the right eye, the eye position information, and the like can be acquired. For example, the controller 130 may be configured to analyze the measurement images to detect the centers of the pupils of the left eye and the right eye, and configured to obtain the pupillary distance using the position information (coordinates). Further, for example, the controller 130 may be configured to analyze the measurement images to detect the centers of the pupils of the left eye and the right eye, and to obtain the eye position information (as an example, the presence or absence and the degree of the squint or the oblique position) based on the relationship between the position information (coordinates) and the bright spots.

In a case where the eye refractive power of the left eye and the eye refractive power of the right eye are acquired regardless of the measurement mode of the subject eye E, the possibility of anisometropia may be determined based on the difference in the eye refractive power.

In the first example and the second example, it is possible to measure the eye refractive power of the subject eye E by at least one of the measurement method different from the photorefraction method and the photorefraction method. Therefore, for example, in a case where both measurement methods are performed on the subject eye E, the first eye refractive power obtained using the method different from the photorefraction method and the second eye refractive power obtained using the photorefraction method may be output in a comparable manner. As an example, the first eye refractive power and the second eye refractive power may be displayed side by side, or may be displayed by switching. The output form is not limited to display, and may be printed or the like.

**Claims**

1. An eye examination device comprising:

   a first objective measurement means configured to objectively measure eye refractive power of at least one of subject eyes using a method different from a photorefraction method; a second objective measurement means configured to objectively measure eye refractive power of at least one of the subject eyes using the photorefraction method; and a controller configured to control at least one of the first objective measurement means and the second objective measurement means to acquire the eye refractive power.

2. The eye examination device according to claim 1,

   wherein the first objective measurement means is configured to project a measurement light flux onto a fundus of one of the subject eyes to measure eye refractive power using the method different from the photorefraction method, and the second objective measurement means is configured to project a measurement light flux onto fundi of both of the subject eyes to measure eye refractive power using the photorefraction method.

3. The eye examination device according to claim 1 or 2,

   wherein the first objective measurement means is an objective measurement means configured to project a measurement light flux onto a fundus of at least one of the subject eyes and to cause a first detector to receive a reflected light flux that is the measurement light flux reflected from the fundus, the first detector being disposed at a fundus conjugate position of one of the subject eyes, and the second objective measurement means is an objective measurement means configured to project a measurement light flux onto a fundus of at least one of the subject eyes and to cause a second detector to receive a reflected light flux that is the measurement light flux reflected from the fundus, the second detector being disposed at a pupil conjugate position of one of the subject eyes.

4. The eye examination device according to claim 2 or 3,

   wherein the first objective measurement means is an objective measurement means configured to project a pattern index, as the measurement light flux, onto the fundus of at least one of the subject eyes and to cause a first detector to receive a reflected light flux that is the measurement light flux reflected from the fundus, and the controller is configured to acquire eye refractive power based on the reflected light flux received by the first detector.

5. The eye examination device according to claim 3,

   wherein the first objective measurement means is an objective measurement means configured to scan the fundus of at least one of the subject eyes with the measurement light flux and to cause the first detector to receive a reflected light flux that is the measurement light flux reflected from the fundus, and the controller is configured to acquire eye refractive power based on a phase difference signal from the first detector.

6. The eye examination device according to any one of claims 1 to 5, further comprising:

   a first alignment means configured to capture at least one of the subject eyes and to adjust a positional relationship between the subject eyes and the first objective measurement means; and a second alignment means configured to capture at least one of the subject eyes at a wider angle than the first alignment means and to adjust a positional relationship between the subject eyes and the second objective measurement means.

7. The eye examination device according to any one of claims 1 to 6, wherein the controller is configured to set one of a first measurement mode using the first objective measurement means and a second measurement mode using the second objective measurement means, based on a switching signal of a measurement mode of eye refractive power of at least one of the subject eyes.

8. The eye examination device according to any one of claims 1 to 7,

   wherein the first objective measurement means includes a light projection optical system configured to project light toward an anterior segment of at least one of the subject eyes, and a measurement light source of the second objective measurement means is also used as a light source of the light projection optical system.

9. The eye examination device according to any one of claims 1 to 8,

wherein the first objective measurement means includes an anterior segment capturing optical system configured to capture an anterior segment of at least one of the subject eyes, and a detector of the second objective measurement means is also used as a detector of the anterior segment capturing optical system.

10. An attachment to be mounted to an eye examination device including a first objective optical system configured to objectively acquire information on a subject eye, the attachment comprising:
a conversion optical system configured to convert the first objective optical system into a second objective optical system configured to objectively measure eye refractive power of the subject eye using a photorefraction method.

11. The attachment according to claim 10, wherein the conversion optical system includes at least one of a plurality of measurement light sources disposed in a meridian direction with respect to a center of an optical axis of the conversion optical system, and a wide-angle lens configured to widen an image capturing angle-of-view of the second objective optical system in a case where the attachment is mounted compared with an image capturing angle-of-view of the first objective optical system in a case where the attachment is not mounted.

12. The attachment according to claim 10 or 11, further comprising:
a distance measurement means configured to measure a distance from the subject eye to the eye examination device.

13. The attachment according to claim 12, wherein the distance measurement means includes an ultrasonic transmission means configured to transmit an ultrasonic wave toward the subject eye, and an ultrasonic reception means configured to receive the ultrasonic wave reflected from the subject eye.

14. An eye examination device to which the attachment according to any one of claims 10 to 13 is capable of being mounted, the eye examination device comprising:

a mounting portion to which the attachment is to be mounted; and
a connection portion configured to electrically connect the eye examination device and the attachment,
wherein the attachment becomes available in a case where the connection portion electrically connects the eye examination device and the attachment.

15. An eye examination program used in an eye examination device including a first objective measurement means configured to objectively measure eye refractive power of a subject eye using a method different from a photorefraction method, and a second objective measurement means configured to objectively measure eye refractive power of the subject eye using the photorefraction method, the eye examination program comprising instructions which, when executed by a processor of the eye examination device, cause the eye examination device to:
control at least one of the first objective measurement means and the second objective measurement means to acquire the eye refractive power.

# FIG. 1

*FIG. 2*

FIG. 3

EP 4 186 412 A1

EP 4 186 412 A1

## FIG. 4

EP 4 186 412 A1

*FIG. 5*

*FIG. 6*

30

## FIG. 7

## FIG. 8

FIG. 9

Europäisches
Patentamt

European
Patent Office

Office européen
des brevets

# EUROPEAN SEARCH REPORT

Application Number

EP 22 20 9800

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/100666 A1 (TAKII MICHIHIRO [JP] ET AL) 2 April 2020 (2020-04-02)<br>* paragraphs [0024] – [0026] *<br>* paragraphs [0077] – [0079] *<br>* paragraphs [0109], [0131], [0133], [0158] *<br>* paragraphs [0167] – [0168] *<br>* paragraphs [0177] – [0178] *<br>* figures 1, 3, 4, 9 *<br>----- | 1-4,6-8, 15 | INV.<br>A61B3/103 |
| X | US 2017/007799 A1 (SAMEC NICOLE ELIZABETH [US] ET AL) 12 January 2017 (2017-01-12)<br>* paragraphs [1818] – [1857] *<br>* figure 19 *<br>----- | 1,5,9,15 | |
| X | WO 2021/037718 A1 (ZEISS CARL VISION INT GMBH [DE]) 4 March 2021 (2021-03-04)<br>* page 25, lines 1-4, 24-32 *<br>* page 42, line 13 – page 43, line 14 *<br>* figures 1, 2 *<br>----- | 1,15 | |
| X | US 2019/117060 A1 (HEGDE PRASANNA [IN] ET AL) 25 April 2019 (2019-04-25)<br>* paragraph [0042] *<br>* paragraphs [0039] – [0047] *<br>* figure 2 *<br>----- | 10-14 | TECHNICAL FIELDS SEARCHED (IPC)<br><br>A61B |

The present search report has been drawn up for all claims

| Place of search | Date of completion of the search | Examiner |
|---|---|---|
| Munich | 24 March 2023 | Gärtner, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 20 9800

This annex lists the patent family members relating to the patent documents cited in the above-mentioned European search report.
The members are as contained in the European Patent Office EDP file on
The European Patent Office is in no way liable for these particulars which are merely given for the purpose of information.

24-03-2023

| Patent document cited in search report | Publication date | Patent family member(s) | | Publication date |
|---|---|---|---|---|
| US 2020100666 A1 | 02-04-2020 | CN | 110960184 A | 07-04-2020 |
| | | EP | 3656286 A1 | 27-05-2020 |
| | | JP | 2020049144 A | 02-04-2020 |
| | | US | 2020100666 A1 | 02-04-2020 |
| US 2017007799 A1 | 12-01-2017 | AU | 2016233268 A1 | 05-10-2017 |
| | | AU | 2016233280 A1 | 12-10-2017 |
| | | AU | 2020202634 A1 | 14-05-2020 |
| | | AU | 2020203809 A1 | 02-07-2020 |
| | | AU | 2021204281 A1 | 22-07-2021 |
| | | AU | 2021229224 A1 | 07-10-2021 |
| | | CA | 2979687 A1 | 22-09-2016 |
| | | CA | 2979811 A1 | 22-09-2016 |
| | | CN | 107530034 A | 02-01-2018 |
| | | CN | 107645921 A | 30-01-2018 |
| | | CN | 113397472 A | 17-09-2021 |
| | | EP | 3270784 A1 | 24-01-2018 |
| | | EP | 3271776 A1 | 24-01-2018 |
| | | HK | 1247804 A1 | 05-10-2018 |
| | | HK | 1249933 A1 | 16-11-2018 |
| | | IL | 281566 A | 31-05-2021 |
| | | IL | 293029 A | 01-07-2022 |
| | | IL | 298199 A | 01-01-2023 |
| | | JP | 6887953 B2 | 16-06-2021 |
| | | JP | 7179910 B2 | 29-11-2022 |
| | | JP | 2018509983 A | 12-04-2018 |
| | | JP | 2018512204 A | 17-05-2018 |
| | | JP | 2021041244 A | 18-03-2021 |
| | | JP | 2021130010 A | 09-09-2021 |
| | | JP | 2023021120 A | 09-02-2023 |
| | | KR | 20170128541 A | 22-11-2017 |
| | | KR | 20170137726 A | 13-12-2017 |
| | | KR | 20210144958 A | 30-11-2021 |
| | | NZ | 773811 A | 01-07-2022 |
| | | NZ | 773812 A | 29-07-2022 |
| | | NZ | 773813 A | 29-07-2022 |
| | | NZ | 773815 A | 01-07-2022 |
| | | NZ | 773816 A | 29-07-2022 |
| | | NZ | 773817 A | 29-07-2022 |
| | | NZ | 773818 A | 29-07-2022 |
| | | NZ | 773819 A | 01-07-2022 |
| | | NZ | 773820 A | 29-07-2022 |
| | | NZ | 773822 A | 29-07-2022 |
| | | NZ | 773823 A | 29-07-2022 |
| | | NZ | 773824 A | 29-07-2022 |
| | | NZ | 773825 A | 29-07-2022 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 22 20 9800

This annex lists the patent family members relating to the patent documents cited in the above-mentioned European search report.
The members are as contained in the European Patent Office EDP file on
The European Patent Office is in no way liable for these particulars which are merely given for the purpose of information.

24-03-2023

| Patent document cited in search report | Publication date | Patent family member(s) | | Publication date |
|---|---|---|---|---|
| | | NZ | 773826 A | 29-07-2022 |
| | | NZ | 773827 A | 29-07-2022 |
| | | NZ | 773831 A | 01-07-2022 |
| | | NZ | 773833 A | 01-07-2022 |
| | | NZ | 773834 A | 01-07-2022 |
| | | NZ | 773836 A | 01-07-2022 |
| | | NZ | 773837 A | 29-07-2022 |
| | | NZ | 773838 A | 29-07-2022 |
| | | NZ | 773839 A | 29-07-2022 |
| | | NZ | 773840 A | 29-07-2022 |
| | | NZ | 773841 A | 01-07-2022 |
| | | NZ | 773842 A | 01-07-2022 |
| | | NZ | 773843 A | 01-07-2022 |
| | | NZ | 773844 A | 01-07-2022 |
| | | NZ | 773845 A | 01-07-2022 |
| | | NZ | 773847 A | 01-07-2022 |
| | | NZ | 773849 A | 01-07-2022 |
| | | US | 2016270656 A1 | 22-09-2016 |
| | | US | 2016287153 A1 | 06-10-2016 |
| | | US | 2017000324 A1 | 05-01-2017 |
| | | US | 2017000325 A1 | 05-01-2017 |
| | | US | 2017000326 A1 | 05-01-2017 |
| | | US | 2017000329 A1 | 05-01-2017 |
| | | US | 2017000330 A1 | 05-01-2017 |
| | | US | 2017000331 A1 | 05-01-2017 |
| | | US | 2017000332 A1 | 05-01-2017 |
| | | US | 2017000333 A1 | 05-01-2017 |
| | | US | 2017000334 A1 | 05-01-2017 |
| | | US | 2017000335 A1 | 05-01-2017 |
| | | US | 2017000337 A1 | 05-01-2017 |
| | | US | 2017000340 A1 | 05-01-2017 |
| | | US | 2017000341 A1 | 05-01-2017 |
| | | US | 2017000342 A1 | 05-01-2017 |
| | | US | 2017000343 A1 | 05-01-2017 |
| | | US | 2017000345 A1 | 05-01-2017 |
| | | US | 2017000454 A1 | 05-01-2017 |
| | | US | 2017000683 A1 | 05-01-2017 |
| | | US | 2017001032 A1 | 05-01-2017 |
| | | US | 2017007111 A1 | 12-01-2017 |
| | | US | 2017007115 A1 | 12-01-2017 |
| | | US | 2017007116 A1 | 12-01-2017 |
| | | US | 2017007122 A1 | 12-01-2017 |
| | | US | 2017007123 A1 | 12-01-2017 |
| | | US | 2017007182 A1 | 12-01-2017 |
| | | US | 2017007450 A1 | 12-01-2017 |
| | | US | 2017007799 A1 | 12-01-2017 |

EPO FORM P0459

For more details about this annex : see Official Journal of the European Patent Office, No. 12/82

**ANNEX TO THE EUROPEAN SEARCH REPORT**

**ON EUROPEAN PATENT APPLICATION NO.**                    EP 22 20 9800

This annex lists the patent family members relating to the patent documents cited in the above-mentioned European search report.
The members are as contained in the European Patent Office EDP file on
The European Patent Office is in no way liable for these particulars which are merely given for the purpose of information.

24-03-2023

| Patent document cited in search report | | Publication date | Patent family member(s) | | Publication date |
|---|---|---|---|---|---|
| | | | US | 2017007843 A1 | 12-01-2017 |
| | | | US | 2017010469 A1 | 12-01-2017 |
| | | | US | 2017010470 A1 | 12-01-2017 |
| | | | US | 2017017083 A1 | 19-01-2017 |
| | | | US | 2019391399 A1 | 26-12-2019 |
| | | | US | 2020041796 A1 | 06-02-2020 |
| | | | US | 2020041797 A1 | 06-02-2020 |
| | | | US | 2020081256 A1 | 12-03-2020 |
| | | | US | 2020409159 A1 | 31-12-2020 |
| | | | US | 2021231959 A1 | 29-07-2021 |
| | | | US | 2021286180 A1 | 16-09-2021 |
| | | | US | 2023004008 A1 | 05-01-2023 |
| | | | WO | 2016149416 A1 | 22-09-2016 |
| | | | WO | 2016149428 A1 | 22-09-2016 |
| WO 2021037718 | A1 | 04-03-2021 | CN | 114340472 A | 12-04-2022 |
| | | | EP | 3782535 A1 | 24-02-2021 |
| | | | EP | 3972478 A1 | 30-03-2022 |
| | | | EP | 3995069 A1 | 11-05-2022 |
| | | | JP | 2022536207 A | 12-08-2022 |
| | | | KR | 20220039769 A | 29-03-2022 |
| | | | US | 2022151484 A1 | 19-05-2022 |
| | | | WO | 2021037718 A1 | 04-03-2021 |
| US 2019117060 | A1 | 25-04-2019 | US | 2019117060 A1 | 25-04-2019 |
| | | | WO | 2017182856 A1 | 26-10-2017 |

EPO FORM P0459

For more details about this annex : see Official Journal of the European Patent Office, No. 12/82

**REFERENCES CITED IN THE DESCRIPTION**

**Patent documents cited in the description**

- JP 2006187483 A
- JP 2012183123 A
- JP 2006149501 A